(19) Europäisches Patentamt
European Patent Office
Office européen des brevets

(11) EP 2 581 370 A1

(12) **EUROPEAN PATENT APPLICATION**
published in accordance with Art. 153(4) EPC

(43) Date of publication:
**17.04.2013 Bulletin 2013/16**

(21) Application number: **11783482.0**

(22) Date of filing: **16.05.2011**

(51) Int Cl.:
***C07D 401/12*** *(2006.01)* ***A61K 31/53*** *(2006.01)*
***A61P 35/00*** *(2006.01)* ***A61P 43/00*** *(2006.01)*

(86) International application number:
**PCT/JP2011/061156**

(87) International publication number:
**WO 2011/145548 (24.11.2011 Gazette 2011/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.05.2010 JP 2010114265**

(71) Applicant: **Astellas Pharma Inc.**
**Tokyo 103-8411 (JP)**

(72) Inventors:
- **SHIMADA, Itsuro**
  **Tokyo 103-8411 (JP)**
- **HIRAKURA, Yutaka**
  **Tokyo 103-8411 (JP)**
- **YAMAZAKI, Kouji**
  **Tokyo 103-8411 (JP)**
- **TAKEGUCHI, Kazuhiro**
  **Tokyo 103-8411 (JP)**
- **UEDA, Norihiro**
  **Tokyo 103-8411 (JP)**

(74) Representative: **HOFFMANN EITLE**
**Patent- und Rechtsanwälte**
**Arabellastrasse 4**
**81925 München (DE)**

(54) **CRYSTALS OF DI(ARYLAMINO)ARYL COMPOUND**

(57) PROBLEM

Provided are crystals of compound A which have properties suitable for industrial production.

MEANS FOR SOLVING

As the results of intensive studies to provide crystals of compound A having an inhibitory activity on the kinase activity of an EML4-ALK fusion protein and a mutant EGFR protein, crystals of compound A were found. Moreover, it was found that A04-type crystal of compound A, from among the aforesaid crystals of compound A, unexpectedly have preferred properties as a drug substance.

EP 2 581 370 A1

**Description**

TECHNICAL FIELD

The present invention relates to a crystal of N-{2-methoxy-4-[4-(4-methylpiperazin-1-yl)piperidin-1-yl]phenyll-N'-[2-(propane-2-sulfonyl)phenyl]-1,3,5-triazine-2,4-diamine (referred to hereinafter as "Compound A").

BACKGROUND ART

It is reported that Compound A represented by formula (I) has a good inhibitory activity against the kinase activity of EML4-ALK fusion protein or mutant EGFR protein and is useful as an active ingredient of a pharmaceutical composition for treating cancer (Patent Document 1).

[Chemical Formula 1]

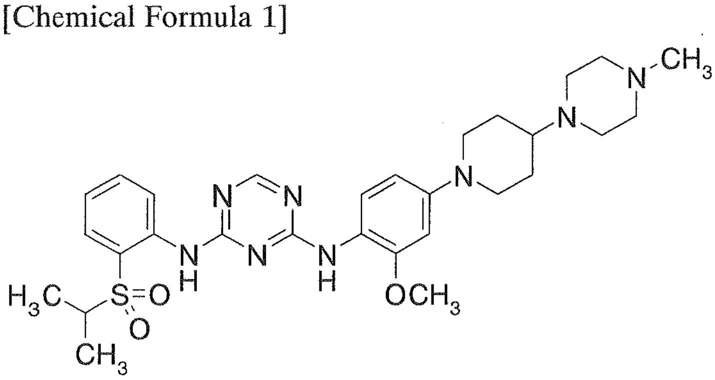

(I)

Example 23 of Patent Document 1 describes a crystal of Compound A whose melting point is 164-165°C. However, there is no disclosure or suggestion on polymorphs of Compound A.

CITATION LIST

PATENT DOCUMENT

Patent Document 1: International Publication WO 2009/008371

SUMMARY OF INVENTION

TECHNICAL PROBLEM

The present invention provides a crystal of Compound A, which is preferable as a drug substance and which has properties suitable for industrial production.

SOLUTION TO PROBLEM

A compound that is used as a drug substance is required to have properties suitable for industrial production and to be in a single form that is easy to handle, because a uniform quality and a stable supply of medicines need to be ensured in the industrial production of medicines.
The present inventors studied polymorphic forms of Compound A intensively, and found to their surprise that differences in specific manufacture conditions induce variations in the generated crystals, and at least 5 types of crystals, namely A01-type, A02-type, A03-type, A04-type, and A05-type, as well as a hydrate are formed for Compound A. Further, the present inventors reached a surprising understanding that the crystal form of Compound A having preferred properties as a drug substance is the A04-type crystal. However, the A04-type crystal was originally obtained only as a mixed crystal with a crystal having a close stability level (especially, A03 crystal) or a solvate, and it was extremely difficult to obtain the A04-type crystal as a single crystal. By many trials and errors of examining the crystallization condition of the A04-type crystal, the present inventors were able to successfully produce the A04-type crystal alone and complete the invention.
In summary, the present invention relates to the A04-type crystal of Compound A that is preferable as a drug substance

and that has properties suitable for industrial production.

The present invention also relates to a pharmaceutical composition comprising A04-type crystal of Compound A. The present invention also relates to a pharmaceutical composition for treating cancer comprising A04-type crystal of Compound A.

The present invention also relates to the use of A04-type crystal of Compound A in manufacturing a pharmaceutical composition for treating cancer, a method for treating cancer comprising administering an effective amount of A04-type crystal of Compound A to a patient, and the use of A04-type crystal of Compound A for treating cancer.

On a side note, the crystal of Compound A described in Example 23 of Patent Document 1 has a melting point of 164-165°C, and it is understood that the crystal is A01-type crystal, A02-type crystal or a mixed crystal thereof based on the comparison of the melting point with the peak temperatures of the crystals of Compound A in the DSC charts in the following drawings.

ADVANTAGEOUS EFFECTS OF INVENTION

The A04-type crystal of Compound A of the present invention has good storage stability and preferred properties as a drug substance; the crystal can be obtained as a single crystal; most importantly, it has good filtering properties and properties suitable for industrial production, hence, the crystal is an extremely useful form in the use of Compound A as a drug substance.

Note that the terms "A01-ty crystal", "'A02-type crystal", "A03-type crystal", "A04-type crystal", and "A05-type crystal" below respectively refer to "A01-type crystal of Compound A", "A02-type crystal of Compound A", "A03-type crystal of Compound A", "A04-type crystal of Compound A", and "A05-type crystal of Compound A".

BRIEF DESCRIPTION OF DRAWINGS

Figure 1 shows a Ruth plot (y axis: $\Delta\theta/\Delta\nu$, x axis: v) of A02-type crystal obtained in the constant pressure filtration experiment.

Figure 2 shows a Ruth plot (y axis: $\Delta\theta/\Delta\nu$, x axis: v) of A04-type crystal obtained in the constant pressure filtration experiment.

Figure 3 shows a solubility graph (y axis: solubility, x axis: temperature) of A02-type crystal, A03-type crystal and A04-type crystal in a 50% acetone aqueous solution.

Figure 4 shows differences in the stability level (y axis: free energy, x axis: temperature) among polymorphic forms, namely A01-type crystal, A02-type crystal, A03-type crystal, A04-type crystal and A05-type crystal.

Figure 5 shows a powder X-ray diffraction pattern of A04-type crystal prepared in Example 2.

Figure 6 shows a DSC chart of A04-type crystal prepared in Example 2.

Figure 7 shows a powder X-ray diffraction pattern of mixed crystals of A01-type crystal and A02-type crystal prepared in Reference Example 1.

Figure 8 shows a DSC chart of mixed crystals of A01-type crystal and A02-type crystal prepared in Reference Example 1.

Figure 9 shows a powder X-ray diffraction pattern of A02-type crystal prepared in Reference Example 2.

Figure 10 shows a DSC chart of A02-type crystal prepared in Reference Example 2.

Figure 11 shows a powder X-ray diffraction pattern of A01-type crystal prepared in Reference Example 3.

Figure 12 shows a DSC chart of A01-type crystal prepared in Reference Example 3.

Figure 13 shows a powder X-ray diffraction pattern of A03-type crystal prepared in Reference Example 4.

Figure 14 shows a DSC chart of A03-type crystal prepared in Reference Example 4.

Figure 15 shows a powder X-ray diffraction pattern of A05-type crystal prepared by Reference Example 5.

Figure 16 shows a DSC chart of A05-type crystal prepared by Reference Example 5.

Figure 17 shows a powder X-ray diffraction pattern of a hydrate of Compound A prepared by Reference Example 6.

Figure 18 shows a DSC chart of a hydrate of Compound A prepared by Reference Example 6.

The measurement conditions applied to the analyses of Figures 5-18 are as follows.

TA Instruments Q-2000, TA Instruments 2910, or TA Instruments Q1000 was used in the measurement for the DSC analysis according to the following conditions: temperature range of measurement: from room temperature to 220°C or higher (modified as necessary); rate of temperature increase: 10°C /min; nitrogen flow rate: 50 mL/min; aluminum sample pan.

RINT-Ultima III or MXP18TAHF22 produced by Mac Science was used in the measurement of powder X-ray diffraction according to the following conditions: X-ray tube: Cu; tube-current: 40 mA or 200 mA; tube-voltage: 40 kV; sampling

width: 0.020°; scanning speed: 3°/min or 4°/min; wavelength: 1.54056 Å; range of measurement diffraction angles (2θ): 2.5-40° or 3-40°.

DESCRIPTION OF EMBODIMENTS

The present invention is described in detail below.
The A04-type crystal of the present invention can be obtained as a single crystal; it excels in filtration properties, and has good solubility, hygroscopicity, stability and/or handling properties. It is also the most stable crystal among the A01-type crystal, the A02-type crystal, the A03-type crystal, the A04-type crystal and the A05-type crystal throughout all temperature ranges. Hence, the A04-type crystal of the present invention is an extremely useful form in the use of Compound A as a drug substance.
Note that no crystal was observed in the crystal screening of Compound A using various solvents other than A01-type crystal, A02-type crystal, A03-type crystal, A04-type crystal, A05-type crystalsand a hydrate of Compound A.
Further, crystals of various salts of Compound A were obtained for assessment, but none of the crystals exhibited preferred properties as a drug substance; 14 types of salts were obtained, specifically monohydrochloride, trihydrochloride, monofumarate, monosulfate, monomesylate, hemifumarate, monotosylate, monophosphate, diphosphate, trihydrobromide, monotartrate, monosuccinate, monomalate, monoglutamate.
The A01-type crystal, the A02-type crystal, the A03-type crystal, the A04-type crystal, the A05-type crystal and a hydrate of Compound A are differentiated by the powder X-ray diffraction measurement and/or DSC analysis (refer to Figures 5-18).
The physicochemical properties of the A04-type crystal of the present invention are shown below.

[1] It has heat absorption peak around 180°C in a DSC analysis (rate of temperature increase: 10°C/min).
[2] It has peaks around 2θ(°)=6.5, 7.9, 12.3, 13.1, 14.4, 15.3, 15.9, 17.0, 17.6, 18.6, 19.0, 19.9, 20.6, 21.0, 21.5, 22.2, 23.2, 23.9, 24.9, 25.9, and 28.8 in a powder X-ray diffraction. Examples of distinctive peaks include peaks around 2θ(°)=6.5, 15.9, 19.9, 23.2 and 24.9. Further, the peak around 2θ(°)=6.5 is a distinctive peak observed only for the A04-type crystal in the A01-type crystal, A02-type crystal, A03-type crystal, A04-type crystal, A05-type crystal and a hydrate of Compound A, under the following analysis conditions.

The measurement conditions for the above analyses are described below.
TA Instruments Q-2000 was used in the measurement for the DISC analysis according to the following conditions: temperature range of measurement: room temperature to 220°C; rate of temperature increase: 10°C/min; nitrogen flow rate: 50 mL/min; aluminum sample pan.
RINT-Ultima III was used in the measurement of powder X-ray diffraction according to the following conditions: X-ray tube: Cu; tube-current: 40 mA; tube-voltage: 40 kV; sampling width: 0.020°; scanning speed: 3°/min; wavelength: 1.54056 Å; range of measurement diffraction angles (2θ): 3-40°.
Note that the spacing of the crystal lattice and the overall pattern are important in identifying crystals based on the nature of the powder X-ray diffraction data, and the relative intensity should not be strictly interpreted, since it can change slightly according to the crystal growth direction, the particle size and the measurement conditions.
Further, the description "around", which takes in account various errors during the measurement, indicates ±3°C for one embodiment and ±2°C for a different embodiment in a DSC analysis and ±2° for one embodiment and ±1° for a different embodiment in a powder X-ray diffraction.

<Production Method>

The A02 crystal can be prepared, for example, by the methods shown in Reference Example 2 and in the Variation of Reference Example 2.
The A04-type crystal of the present invention can be obtained by the solvent-mediated transformation from an A02-type crystal. Specifically, the A04-type crystal can be prepared by suspending and stirring the A02-type crystal in a solvent under heat, at 40°C or higher for one embodiment, at 50°C or higher for another embodiment and at 60°C or higher for yet another embodiment. Examples of solvent used herein are not particularly limited and include 50% acetone aqueous solution, methyl ethyl ketone, and acetone. In one embodiment, 50% acetone aqueous solution or methyl ethyl ketone is used.
The A04-type crystal of the present invention can be prepared by heating and dissolving Compound A in a solvent, then, stirring the product under heat at 40°C or higher for one embodiment, and at 60°C or higher for another embodiment. Examples of solvent used herein are not particularly limited and include 50% ethanol aqueous solution and methyl ethyl ketone.
Further, a seed crystal of the A04-type crystal can be added to produce the A04-type crystal of the present invention

efficiently and with high reproducibility.
Note that Compound A and other starting compounds can be prepared by the method in Patent Document 1 or methods that are obvious to a person skilled in the art.

The pharmaceutical composition comprising the crystal of Compound A of the present invention as an active ingredient can be prepared by commonly used methods using excipients, that is, excipients for pharmaceutical agents or carriers for pharmaceutical agents, commonly used in the art.
The pharmaceutical composition can be administered by either oral administration of tablets, pills, capsules, granules, powders, liquid drugs and the like, or by parenteral administration through intraarticular, intravenous, intramascular or other injections, suppositories, eye drops, eye ointments, endermic liquid formulations, ointments, endermic patches, transmucosal liquid formulations, transmucosal patches, inhalants and the like.

As the solid composition for oral administration, tablets, powders, granules and the like are used. In such a solid composition, the crystal of Compound A is mixed with at least one inert excipient. In accordance with common methods, the composition may contain inert additives such as lubricants, disintegrants, stabilizers, and solubilizing agents. As occasion demands, the tablets or pills may be coated with a sugar coating or a film of a gastric or enteric substance.
Formulations for external use include ointments, plasters, creams, jellies, cataplasms, sprays, lotions, eye drops, eye ointments, and the like. The formulations contain generally used ointment bases, lotion bases, aqueous or non-aqueous liquid preparations, suspensions, emulsions, and the like.

Regarding the transmucosal formulations such as an inhalation, a transnasal formulation, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of prescribed mixture, or as a solution or suspension in combination with a pharmaceutically acceptable carrier, using a conventionally known device or sprayer, such as a measured administration inhalation device, and the like. The dry powder inhaler or the like may be for single or multiple administration use, and a dry powder or a powder-containing capsule may be used. Alternatively, this may be in a form such as a pressurized aerosol spray which uses an appropriate propellant, for example, a suitable gas such as chlorofluoroalkane, carbon dioxide, or the like.

Generally, in the case of oral administration, the daily dose is from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably administered from about 0.0001 to 10 mg/kg per body weight, once a day or two or more times a day. In addition, a transmucosal formulation is administered at a dose from about 0.001 to 100 mg/kg per body weight, once a day or two or more times a day. The dose is appropriately decided in response to the individual case by taking the symptoms, the age, the gender, and the like into consideration.

The crystal of Compound A of the present invention can be used in combination with various therapeutic or prophylactic agents for diseases against which Compound A would be effective. In general, when an antitumor agent is administered alone during chemotherapy for tumor, particularly malignant tumor, the antitumor agent has a limit in its effect in terms of side effects and the like, and thus often fails to produce a sufficient antitumor effect. For this reason, in clinical cases, multidrug therapy is used in which two or more drugs with different mechanisms of action are combined. By combining antitumor agents with different mechanisms of action, this combination therapy aims to reduce side effects and/or enhance the desired antitumor effect, for example, 1) to reduce the number of non-sensitive cell population, 2) to prevent or delay the development of drug resistance, 3) to disperse toxicity by combination of drugs with different toxicity levels, and the like. In such combination therapy, drugs may be administered simultaneously or separately in succession or at desired time intervals. Formulations for simultaneous administration may be in either mixed or separate form.
Drugs which can be combined include chemotherapeutics (e.g., alkylating agent, antimetabolite, and the like), immunotherapeutic agents, hormonal therapeutic agents, and cell growth factor inhibitors, more specifically drugs such as cisplatin, carboplatin, paclitaxel, docetaxel, gemcitabine, irinotecan, vinorelbine, bevacizumab, and the like.

EXAMPLES

The present invention is described in detail below by the Examples, without being limited in scope thereby, and the embodiment of the present invention can be modified as necessary by methods obvious to a person skilled in the art.
In addition, preparation methods of a mixed crystal of A01-type crystal and A02-type crystal is provided as Reference Example 1, the preparation method of A02-type crystal is provided as Reference Example 2 and a Variation of Reference Example 2, the preparation method of A01-type crystal is provided as Reference Example 3 and a Variation of Reference Example 3, the preparation method of A03-type crystal is provided as Reference Example 4, the preparation method of A05-type crystal is provided as Reference Example 5 and a Variation of Reference Example 5 and the preparation

method of a hydrate of Compound A is provided as Reference Example 6.
The nuclear magnetic resonance spectrum (NMR) was measured using JNM-AL400 produced by JEOL, Ltd. in deuterated chloroform (CDCL3) using tetramethylsilane (TMS) as an internal standard.

<Example 1>

To 5 g of A02-type crystal, 50 mL of methyl ethyl ketone was added and the mixture was stirred under suspension at about 50°C for 5 days. Then, the mixture was slowly cooled to 25°C, the precipitated solid was collected by filtration, and the solid was washed with 5 mL of methyl ethyl ketone and subsequently dried to obtain 3.88 g of A04-type crystal.

<Example 2>

To 13 kg of A02-type crystal, 78 L of 50% acetone aqueous solution preheated to about 60°C and 1.3 g of A04-type crystal were added, and the mixture was stirred under suspension at about 60°C for about 2.5 hours. Then, 26 L of 50% acetone aqueous solution was added and the mixture was stirred for about 21.5 hours. Then, the mixture was slowly cooled to 25°C, the precipitated solid was collected by filtration, and the solid was washed with 78 L of 50% acetone aqueous solution and subsequently dried to obtain 11.03 kg of A04-type crystal.

$^1$H-NMR (CDCL3, 400MHz)

δ(ppm)=1.31(d, 6H, J=6.8Hz), 1.58-1.80(m, 4H), 1.90-2.04(m, 2H), 2.16-2.84(m, 12H), 3.18-3.32(m, 1H), 3.66-3.76(m, 2H), 3.88(s, 3H), 6.48-6.60(m, 2H), 7.18-7.26(m, 1H), 7.50-7.72(m, 2H), 7.86-7.92(dd, 1H, J=1.2Hz, J=7.6Hz), 8.06-8.16(m, 1H), 8.28-8.48(m, 1H), 8.48-8.62(m, 1H), 9.28(s, 1H)

<Example 3>

To 10.0 g of Compound A, 200 mL of methyl ethyl ketone was added and dissolved under heating. Then, 10 mg of A04-type crystal was added at about 70°C and stirred at about 60°C for 7 hours. Then, the mixture was slowly cooled to 25°C, the precipitated solid was collected by filtration, and the solid was washed with 20 mL of methyl ethyl ketone and subsequently dried to obtain 7.6 g of A04-type crystal.

<Example 4>

To 10.0 g of Compound A, 210 mL of methyl ethyl ketone was added and dissolved under heating, then 110 mL of methyl ethyl ketone was removed by condensation under normal pressure.
Then, 1 mg of A04-type crystal was added at about 70°C and stirred at about 70°C for 1 hour. After 80 mL of n-heptane was added at about 70°C and the mixture was stirred for 30 minutes, the mixture was slowly cooled to 25°C. The precipitated solid was collected by filtration, and then the solid was washed with a mixture of 10 mL of methyl ethyl ketone and 10 mL of n-heptane and subsequently dried under reduced pressure to obtain 9.15 g of A04-type crystal.

$^1$H-NMR (CDCL3, 400MHz)

δ(ppm)=1.31(d, 6H, J=6.8Hz), 1.57-1.78(m, 4H), 1.90-2.00(m, 2H), 2.22-2.80(m, 12H), 3.20-3.33(m, 1H), 3.60-3.80(m, 2H), 3.88(s, 3H), 6.50-6.60(m, 2H), 7.18-7.30(m, 1H), 7.50-7.70(m, 2H), 7.80-7.92(dd, 1H, J=0.8Hz, J=5.6Hz), 8.00-8.20(m, 1H), 8.30-8.48(m, 1H), 8.48-8.60(m, 1H), 9.28(s, 1H)

<Reference Example 1>

To a mixture of 30.0 g of 4-chloro-N-[2-(propane-2-sulfonyl)phenyl]-1,3,5-triazin-2-amine and 450 mL of ethanol, 19 mL of methanesulfonic acid was added and the mixture was stirred under room temperature for 15 minutes, then 29.2 g of 2-methoxy-4-[4-(4-methylpiperazin-1-yl)piperidin-1-yl]aniline was added and the mixture was stirred at 100°C for 5 hours. The reaction mixture was cooled, to the mixture was added 900 mL of diethyl ether, and the precipitated solid was collected by filtration. The collected solid was dissolved in 300 mL of water, to which a saturated sodium hydrogen carbonate aqueous solution was added to form a mixture of pH8, then it was extracted 3 times using 300 mL of ethyl acetate, respectively. The extract was washed with water and saturated saline, dried with anhydrous sodium sulfate, and then the solvent was distilled out under reduced pressure. The residue was purified by silica gel column chromatography (developing solvent: chloroform/methanol/concentrated ammonia aqueous solution=100/1/0→50/1/0→30/1/0→30/1/0.1→20/1/0.1), and the resultant solid was collected by filtration and washed with

ethanol to obtain 27.94 g of a mixed crystal of A01-type crystal and A02-type crystal.

$^{1}$H-NMR (CDCL3, 400MHz)

δ(ppm)=1.31(d, 6H, J=8.0Hz), 1.59-1.78(m, 4H), 1.90-2.01(m, 2H), 2.22-2.80(m, 12H), 3.19-3.33(m, 1H), 3.65-3.76(m, 2H), 3.88(s, 3H), 6.49-6.60(m, 2H), 7.17-7.30 (m, 1H), 7.50-7.70(m, 2H), 7.84-7.92(dd, 1H, J=1.6Hz, J=8.0Hz), 8.04-8.17(m, 1H), 8.30-8.48(m, 1H), 8.48-8.62(m, 1H), 9.28(s, 1H)

<Reference Example 2>

Compound A (100 mg) was dissolved under heating to about 2 mL of acetone and cooled, then the precipitated solid was collected by filtration to obtain 65 mg of A02-type crystal.

$^{1}$H-NMR (CDCL3, 400MHz)

δ(ppm)=1.31(d, 6H, J=8.0Hz), 1.56-1.78(m, 4H), 1.91-2.02(m, 2H), 2.22-2.80(m, 12H), 3.18-3.33(m, 1H), 3.63-3.77(m, 2H), 3.88(s, 3H), 6.47-6.63(m, 2H), 7.17-7.32 (m, 1H), 7.50-7.73(m, 2H), 7.84-7.92(m, 1H), 8.04-8.17(m, 1H), 8.29-8.48(m, 1H), 8.48-8.65(m, 1H), 9.29(s, 1H)

<Variation of Reference Example 2>

To about 400 L of a methyl ethyl ketone solution containing 29.34 kg of 4-chloro-N-[2-(propane-2-sulfonyl) phenyl]-1,3,5-triazin-2-amine, 60.6 kg of 2-methoxy-4-[4-(4-methylpiperazin-1-yl)piperidin-1-yl]aniline tris(trifluoroacetate) was added and the mixture was stirred at about 65°C for about 3 hours. Then, a mixture of 19.1 kg of sodium chloride, 15.8 kg of sodium hydroxide and 191 kg of water was added, and an organic layer was collected. Then, the organic layer was washed twice by a mixture of 38.1 kg of sodium chloride and 191 kg of water, respectively, followed by condensation of the resultant organic layer under reduced pressure at 50°C until the liquid was about 56 L. Then, 75 kg of acetone was added and condensation under reduced pressure was conducted at 50°C until the liquid was about 56 L, and the same procedure was conducted again. Then, 32 kg of acetone was added, and the mixture was stirred at about 40°C for 1 hour, followed by an addition of 83 kg of isopropyl acetate at around same temperature and 1 hour of stirring. Then, the mixture was slowly cooled until it reached about 0°C, the precipitated solid was collected by filtration, and the solid was washed with a mixture of 15 kg of acetone and 17 kg of isopropyl acetate, and with 33 kg of isopropyl acetate, and then dried to obtain 26.6 kg of A02-type crystal.

Note that 2-methoxy-4-[4-(4-methylpiperazin-1-yl)piperidin-1-yl]aniline tris(trifluoroacetate) used in Reference Example 2 can be prepared by methods obvious to a person skilled in the art, the below described method or other methods.

<Preparation of 2-methoxy-4-[4-(4-methylpiperazin-1-yl)piperidin-1-yl]aniline tris(trifluoroacetate) salt>

After 39.8 kg of 1-[1-(3-methoxy-4-nitrophenyl)piperidin-4-yl]-4-methylpiperazine, 6.0 kg of 10% palladium carbon and 354.5 kg of tetrahydrofuran were mixed, the mixture was stirred under hydrogen pressure of about 0.1 MPa at about 25°C for about 5 hours. Then, the temperature was raised to about 35°C, and palladium carbon was removed by filtration and washed with 68.0 kg of tetrahydrofuran. After 42.2 kg of trifluoroacetic acid was added to the resultant filtrate, 4.0 g of 2-methoxy-4-[4-(4-methylpiperazin-1-yl)piperidin-1-yl]aniline tris(trifluoroacetate) crystals were added and the mixture was stirred at about 35°C for about 1 hour, then 221 kg of n-heptane was added at about 20°C. The precipitated solid was collected by filtration and washed with a mixture of 62.9 kg of tetrahydrofuran and 34.3 kg of n-heptane, then the resultant solid was dried to obtain 71.2 kg of 2-methoxy-4-[4-(4-methylpiperazin-1-yl)piperidin-1-yl] aniline tris(trifluoroacetate) crystals.

$^{1}$H-NMR (DMSO-d6, 400MHz)

δ(ppm)=1.67(m, 2H), 2.06(m, 2H), 2.79(s, 3H), 2.8-3.9(m, 18H), 3.88(s, 3H), 6.62(d, 1H, J=8.4Hz), 6.79(s, 1H), 7.11(d, 1H, J=8.4Hz)

<Variation of Preparation of 2-methoxy-4-[4-(4-methylpiperazin-1-yl)piperidin-1-yl]aniline tris(trifluoroacetate)>

Isopropyl alcohol (57 mL) was added to dissolve 5.7 g of 2-methoxy-4-[4-(4-methylpiperazin-1-yl)piperidin-1-yl]aniline, then 6.62 g of trifluoroacetic acid was added and the mixture was cooled to 0°C. The precipitated solid was

collected by filtration and dried to obtain 11.65 g of 2-methoxy-4-[4-(4-methylpiperazin-1-yl)piperidin-1-yl]aniline tris (trifluoroacetate) crystals.

<Reference Example 3>

A02-type crystal was heated to 150°C at 10°C/min in a DSC furnace, then cooled to room temperature at 10°C/min to prepare A01-type crystal.

<Variation of Reference Example 3>

Acetonitrile (200 mL) was added to 5.0 g of Compound A and dissolved with heating under reflux. The mixture was rapidly cooled to room temperature and the precipitated solid was collected by filtration. The solid was dried to obtain 1.56 g of A01-type crystal.

<Reference Example 4>

Ethanol/water (9:1) (2 mL) was added to about 50 mg of Compound Aand dissolved with heating under reflux. After cooling, the mixture was stirred in an open system for 2 days, then a lid was placed on the system and the mixture was further stirred for 3 days. The precipitated solid was collected by filtration and 21 mg of A03-type crystals were obtained.

<Reference Example 5>

A mixed solvent of 65 mL of ethanol and 35 mL of water was added to 10.0 g of Compound A and dissolved under heating at about 75°C. Then, 30 mL of water was added to the mixture and the mixture was left to cool. A05-type crystal (10 mg) was added at about 30°C, and the mixture was cooled to 25°C, then the precipitated solid was collected by filtration and washed with 20 mL of 50% ethanol aqueous solution and dried to obtain 6.9 g of Compound A crystal. The crystal (1.0338 g) was dried at 50°C under reduced pressure for 7 days to obtain 0.9760 g of A05-type crystal.

<Alternative of Reference Example 5>

A mixed solvent of 3.25 mL of ethanol and 3.25 mL of water was added to 500 mg of Compound A and dissolved under heating at about 80°C, then the mixture was cooled. A04-type crystal (of about 1 spatula) was added at a temperature around room temperature and stirred overnight. Then, the solid was collected by filtration and dried to obtain 340 mg of A05-type crystal.

<Reference Example 6>

A mixed solvent of 65 mL of ethanol and 35 mL of water was added to 10.0 g of Compound A and dissolved under heating at about 75°C, then 30 mL of water was added to the mixture and the mixture was cooled. A05-type crystal (10 mg) was added at about 30°C and the mixture was cooled to 25°C, then the precipitated solid was collected by filtration and washed with 20 mL of 50% ethanol aqueous solution and dried to obtain 6.9 g of Compound A crystal. The crystal (0.8858 g) was stored for about 7 days in a desiccator together with a beaker of water to obtain 0.9441 g of a hydrate of Compound A.

The effects of A04-type crystal of the present invention are shown below. Note that "R.H." represents relative humidity.

<Test Example 1> Evaluation of Storage Stability

(i) Long Term Stability Test

A04-type crystal samples were separately stored for 3 months under (1) 40°C/75% R.H., (2) 60°C/uncontrolled humidity, and (3) 25°C/uncontrolled humidity/D65 lamp (1000 lux) illumination (2 months under condition (3)), and the change in the crystal form of the samples before and after storage was measured by powder X-ray diffraction and DSC analysis and the change in the purity of the same was confirmed by HPLC measurement (using HPLC Condition 1 or HPLC Condition 2 shown below as the mearuement conditions).

The result showed no significant change in the crystal form or the purity for any of the conditions, and A04 crystal was confirmed to be crystal with good physiochemical stability.

(HPLC Condition 1) Mobile phase: (Liquid A) 0.8 g/L ammonium hydrogencarbonate aqueous solution (adjusted to pH10 by ammonia water (28)), (Liquid B) methanol, gradient% Liquid B: 35% (0 min)/linear 70% (0-50 min)/70% (50-70 min), column: XBridge Shield RP18, 4.6x150 mm, particle size 5 μm (produced by Waters), flow rate: 1 mL/min, column temperature: 40°C, detection: UV 214 nm.
(HPLC Condition 2) Mobile phase: (Liquid A) 2.0 g/L ammonium hydrogencarbonate aqueous solution (adjusted to pH9 by ammonia water (28)), (Liquid B) methanol, gradient% Liquid B: 30% (0 min)/linear 70% (0-50 min)/70% (50-70 min), column: XBridge Shield RP18, 4.6x150 mm, particle size 5 μm (produced by Waters), flow rate: 1 mL/min, column temperature: 30°C, detection: UV 214 nm.

(ii) Hygroscopicity Test A

About 0.5 g of A04-type crystal was weighed into an (open) weighing bottle with a known mass to obtain a precise mass, then the weighing bottle was placed in a desiccator adjusted to a relative humidity of 93% by $KNO_3$ saturated aqueous solution and stored at 25°C for 7 days before measuring the change in mass.
The result showed no significant change in mass and demonstrated that A04 crystal does not exhibit hygrocopicity.
In addition, the change in the crystal form of the samples before and after storage is measured by powder X-ray diffraction and DSC analysis and the change in the purity of the same can be confirmed by HPLC measurement.

(iii) Hygroscopicity Test B

The water absorption/dehydration behavior of A04-type crystal was assessed using water balance measuring apparatus SGA-X100 (VTI) under the following conditions. Temperature: 25°C, measurement range: 5-95% R.H., measurement interval: 5%.
The resulting weight change of lower than 0.1% through water absorption and dehydration in the humidity range of 5-95% R.H. showed that A04-type crystal does not exhibit hygroscopicity.
The above results of Test Example 1 clearly show that A04-type crystal has properties preferable as a drug substance, and it is an extremetly useful form in the use of Compound A as a drug substance.

<Test Example 2> Assessment of Filterability

Slurries of A02-type crystal prepared by a method similar to that of the Variation of Reference Example 2 and A04-type crystal prepared by a method similar to that of Example 2 were used to perform constant pressure filteration test, and the cake-average filteration specific resistance $\alpha_m$ was obtained by constant pressure filteration formula of Ruth shown in formula (1) to assess filterability. Note that the slurry used in the present test is the crystallization slurry immediately before the crystals are collected by filtration in the preparation of the crystals.

[Formula 1]

$$\frac{\Delta\theta}{\Delta v} = \frac{\alpha_m c\mu}{x\Delta P} v + \frac{R_m \mu}{\Delta P} \qquad (1)$$

(wherein, θ is the filtration time [sec], ν is the integral filtrate amount per unit filtration area [$m^3/m^2$], $\alpha_m$ is the cake average specific resistance [m/kg], c is the solid content suspension density [$kg/m^3$], μ is the filtrate viscosity [Pa · s], x is the filtrate/slurry volume ratio, ΔP is the applied pressure [Pa], $R_m$ is the filter resistance [$m/m^2$]. The filter resistance $R_m$ is the resistance of filters such as filter cloth or sintered wire gauze used in the filtration device, and shows approximately a constant value for the same filtration condition.
The calculation of cake average specific resistance $\alpha_m$ [m/kg] was performed by plotting (Ruth plot) ν [$m^3/m^2$] and Δθ/Δν [sec/m], obtaining the filtration constant B from the inclination of the obtained line, and inserting the parameters shown in the filtration condition row of Table 1 to formula (2).

[Formula 2]

$$B = \frac{\alpha_m c\mu}{x\Delta P} \qquad (2)$$

The filtration condition, filtration constant B and cake average specific resistance $\alpha_m$ of A02-type crystal and A04-type crystal were respectively shown in Table 1. Further, the result of the Ruth plot of A02-type crystal and A04-type crystal were respectively shown in Figures 1 and 2.

[Table 1]

| Filteration Condition | A02-type crystal | A04-type crystal |
|---|---|---|
| Applied Pressure $\Delta P$ | 100 kPa | 100 kPa |
| Solid Content Suspension Density c | 134 kg/$m^3$ | 124 kg/$m^3$ |
| Filterate Viscosity $\mu$ | 0.47 mPa · s | 1.8 mPa · s |
| Filteration Area | 4.1 $cm^2$ | 5.7 $cm^2$ |
| Filterate/Slurry Volume Ratio x | 0.933 | 0.858 |
| Filtration Constant B | 81406 | 2201.7 |
| Cake Average Specific Resistance $\alpha_m$ | $1.31\times10^{11}$ m/kg | $8.43\times10^{8}$ m/kg |

The cake average specific resistance of the A02-type crystal was $1.31\times10^{11}$ m/kg, whereas that of A04-type crystal was $8.43\times10^{8}$ m/kg, and the value for the A02-type crystal was about 155 times as large as that for the A04-type crystal. The filtration time $\theta$ for constant pressure filtration can be obtained by formula (3).

[Formula 3]

$$\theta = \frac{\mu v}{2\Delta P}\left(\alpha_m cv / x + 2R_m\right) \qquad (3)$$

That is to say that when the constant pressure filtration is performed for A02-type crystal and A04-type crystal under the same condition, A02-type crystal will require filtration time about 155 times as long as that of the A04-type crystal, since the filtration time is proportional to $\alpha_m$ (Note that the filter resistance $R_m$, which depends only on the filter used in filtration, is ignored herein to assess the filterability of the crystals).

The above assessments show that the filterability of A04-type crystal prepared in Example 2 is significantly higher than that of A02-type crystal prepared in the Variation of Reference Example 2.

It is inappropriate to make generalizations since the filtration time varies by the production amount and the filtration device to be used, but it is normally preferable to have a cake average specific resistance of approximately $10^8$ m/kg or lower for productions having a scale in the order of ten to the order of a hundred kg. Hence, there is a great need to improve the filterability of the crystal when the A02-type crystal having a cake average specific resistance that is 100 times the preferable cake average specific resistance or higher is used in industrial production.

It is quite effective to let crystals grow large in order to improve crystal filterability. General methods for inducing crystals to grow large include a method of adding a seed crystal at a high temperature for slow, time consuming growth, or a method of precipitating crystals then raising the temperature again to selectively dissolve microcrystals. However, it is extremely difficult to singly obtain A-02 type crystal in a large size, because parts of A02-type crystal readily transform to A03-type crystal or the most stable A-04 type crystal, and form A03-type crystal, A04-type crystal, or mixed crystals thereof before growing sufficiently as A02-type crystal.

Accordingly, it is at least necessary to perform crystallization under conditions inducing rapid nucleation and obtain the crystal before it transforms in order to stably obtain the A02-type crystal alone. Such conditions inducing rapid nucleation will not allow crystals to grow sufficiently to produce large crystals, so it will be difficult to obtain A02-type crystal having good filterability.

Further, a bad filterability of crystals leads to low washing effects in the washing step using solvents; hence, there is concern of the crystallization mother liquor sticking to the crystals, resulting in the introduction of impurities and a decrease in purity.

<Test Example 3> Assessment of the Amount of Impurities

A02-type crystal prepared by a method similar to a Variation of Reference Example 2 and A04-type crystal prepared by a method similar to Example 2 and Example 4 were subjected to HPLC measurement to assess the amount

of impurities (using the above HPLC Condition 1 or HPLC Condition 3 shown below as the mearuement condition). The result showed the total amount of impurities of A04-type crystal prepared by a method similar to Example 2 and Example 4 to be lower than 1%. In contrast, the total amount of impurities of A02-type crystal prepared by a method similar to a Variation of Reference Example 2 was 2.42%, indicating that the A02-type crystal contain more impurities than A04-type crystal.
In an industrial scale production of A02-type crystal, methods other than the Variation of Reference Example 2 do not allow the stable production of A02-type crystal alone. Hence, it is considered that the amount of impurities in the crystallization mother liquor is also involved with the control of A02-type crystal (it is confirmed that the crystals readily transform to A03-type crystal, A04-type crystal, or their mixed crystals when the amount of impurities in the crystallization mother liquor is small, as mentioned above). That is, the stable production of A02-type crystal alone at an industrial scale requires a given amount of impurities to exist in the crystallization mother liquor, and consequently, much impurity will be contained in the A02-type crystal; hence, it is concluded that industrially produced A02-type crystal contain a large amount of impurities.
(HPLC Condition 3) Mobile phase: 0.1% perchloric acid aqueous solution/acetonitrile = 3:1 (0-30 min), column: L-column 2 ODS, 4.6x150 mm, particle size 5 μm (produced by Chemical Evaluation and Research Institute), flow rate: 1 mL/min, column temperature: 40°C, detection: UV 225 nm.

<Test Example 4> Assessment of the Stability Level

(i) Solubility in 50% Acetone aqueous solution

The solubility of A02-type crystal, A03-type crystal and A04-type crystal was measured for 50% acetone aqueous solution. The result is shown in Table 2 and Figure 3. The solubility is lowest for A04-type crystal at all temperatures. The result showed that the thermodynamic stability level of the crystal of Compound A around room temperature is A04-type crystal>A03-type crystal>A02-type crystal.

[Table 2]

| Temperature | Solubility | | |
|---|---|---|---|
| | A02-type crystal | A03-type crystal | A04-type crystal |
| 25°C | 13.6 g/L | 5.3 g/L | 4.5 g/L |
| 40°C | 17.7 g/L | 8.5 g/L | 8.0 g/L |
| 55°C | 30.8 g/L | 16.1 g/L | 13.0 g/L |

(ii) Study of Solvent-mediated Transformation

A05-type crystal was subjected to slurry agitation under room temperature and its solvent-mediated transformation was studied. It was consequently confirmed that A05-type crystal was transformed to A04-type crystal after 1 minute of agitation in methyl ethyl ketone under room temperature. This showed that the thermodynamic stability level of the crystal of Compound A under room temperature is A04-type crystal>A05-type crystal, since the solvent-mediated transformation results in a transformation to a more stable form.

(iii) DSC Analysis

The following observation can be made from the result of the DSC analyses of A01-type crystal, A02-type crystal, A03-type crystal, A04-type crystal and A05-type crystal shown in the drawings.

(a) The absorption of heat around 142°C in the DSC analysis result of Figure 10 shouws the transformation from A02-type crystal to A01-type crystal. A01-type crystal and A02-type crystal can be transformed into each other, and A02-type crystal is thermodynamically more stable under room temperature.
(b) From the comparison of A01-type crystal (melting point: 164°C, heat of melting: 81 J/g (Figure 12)) and A05-type crystal (melting point: 89°C, heat of melting: 34 J/g (Figure 16)), A01-type crystal and A05-type crystal are in a monotropic relation based on the principle of heat of melting, and the A01-type crystal is thermodynamically more stable than A05-type crystal in all temperature range.
(c) From the comparison of A04-type crystal (melting point: 180°C, heat of melting: 95 J/g (Figure 6)) and A01-type crystal (melting point: 164°C, heat of melting: 81 J/g (Figure 12)), A04-type crystal and A01-type crystal are in a

monotropic relation based on the principle of heat of melting, and the A04-type crystal is thermodynamically more stable than A01-type crystal in all temperature range.

Hence, the thermodynamic stability under room temperature is A02-type crystal>A01-type crystal, and also A04-type crystal>A01-type crystal>A05-type crystal as shown by (a) to (c) above.

The results of (i) to (iii) above show that the thermodynamic stability level under room temperature is A04-type crystal>A03-type crystal>A02-type crystal>A01-type crystal>A05-type crystal. This result and the comparison of the melting peak temperatures of A01-type crystal, A02-type crystal, A03-type crystal, A04-type crystal and A05-type crystal show that A04-type crystal is in monotropic relation to A01-type crystal, A02-type crystal, A03-type crystal and A05-type crystal, and it exhibits the highest melting peak temperature (A01-type crystal: 164°C, A02-type crystal: transformation point to A01-type crystal 142°C, A03-type crystal: 171°C, A04-type crystal: 180°C, A05-type crystal: 89°C), so it is the most stable crystal of the crystal forms of Compound A in all temperature range. The schematic diagram showing the interrelation of the stability levels of the crystals based on the above result is presented as Figure 4.

There are cases in which it is preferable to provide the drug substance in the most stable crystal form to ensure a uniform quality and a stable supply of medicines. A well known example is the ritonavir case. Ritonavir was commercially supplied as the metastable crystal, but one day, a more stable crystal form suddenly appeared, and the commercial production of the metastable form crystal became impossible ever since. To prevent such a risk, there is currently a high demand for drug substances to be supplied in the most stable crystal form.

A04-type crystal of the present invention is considered the most stable form of crystal in all temperature range, so there is low production risk of the crystals in the desired crystal form suddenly vanishing by the rise of the industrial production of the most stable form of crystal, as the ritonavir case. Further, it can be stably supplied as a single crystal, and particularly, it has good filterability and properties suitable for industrial production, so it is an extremely useful form in the use of Compound A as a drug substance.

On the other hand, A01-type crystal and A02-type crystal are a metastable crystal, so they require strict control of the crystallization condition for stable production, and they hold a production risk of the crystal forms suddenly vanishing as in the above ritonavir case.

Further, A02-type crystal has a stronger tendency to take in impurities in the crystal than A04-type crystal, because it is at least necessary to perform crystallization under conditions inducing rapid nucleation and obtain the crystal before it transforms to A04-type crystal or A03-type crystal in order to stably obtain the A02-type crystal as a single crystal, as mentioned above.

As shown above, restricting the introduction of impurities is extremely difficult for A02-type crystal compared to A04 crystal, because the former crystal tends to take in impurities and the washing effects in the washing step is low due to its filterability, as mentioned above.

INDUSTRIAL APPLICABILITY

A04-type crystal of the present invention is extremely useful in the use of Compound A as a drug substance, since A04-type crystal has good storage stability and preferred properties as a drug substance, and it can be obtained as a single crystal, and particularly, it has good filterability and properties suitable for industrial production.

## Claims

**1.** A crystal of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine having a heat absorbing peak around 180°C in a DSC analysis (rate of temperature increase: 10°C/min).

**2.** A crystal of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine having a peak around 2θ(°) = 6.5 in a powder X-ray diffraction (tube: Cu).

**3.** The crystal according to Claim 2 having peaks around 2θ(°) = 6.5, 15.9, 19.9, 23.2 and 24.9 in a powder X-ray diffraction (tube: Cu).

**4.** The crystal according to Claim 3 having peaks around 2θ(°) = 6.5, 7.9, 12.3, 13.1, 14.4, 15.3, 15.9, 17.0, 17.6, 18.6, 19.0, 19.9, 20.6, 21.0, 21.5, 22.2, 23.2, 23.9, 24.9, 25.9, and 28.8 in a powder X-ray diffraction (tube: Cu).

**5.** The crystal according to Claim 1 having a peak around 2θ(°) = 6.5 in a powder X-ray diffraction (tube: Cu).

**6.** A pharmaceutical composition comprising a crystal according to any one of Claims 1-5 as an active ingredient.

**7.** The pharmaceutical composition according to Claim 6 which is a pharmaceutical composition for treating cancer.

**8.** A use of the crystal according to any one of Claims 1-5 in manufacturing a pharmaceutical composition for treating cancer.

**9.** A method for treating cancer comprising administering an effective dosage of the crystal according to any one of Claims 1-5 to a patient.

**10.** The crystal according to any one of Claims 1-5 for treating cancer.

**11.** A use of the crystal according to any one of Claims 1-5 for treating cancer.

[Figure 1]

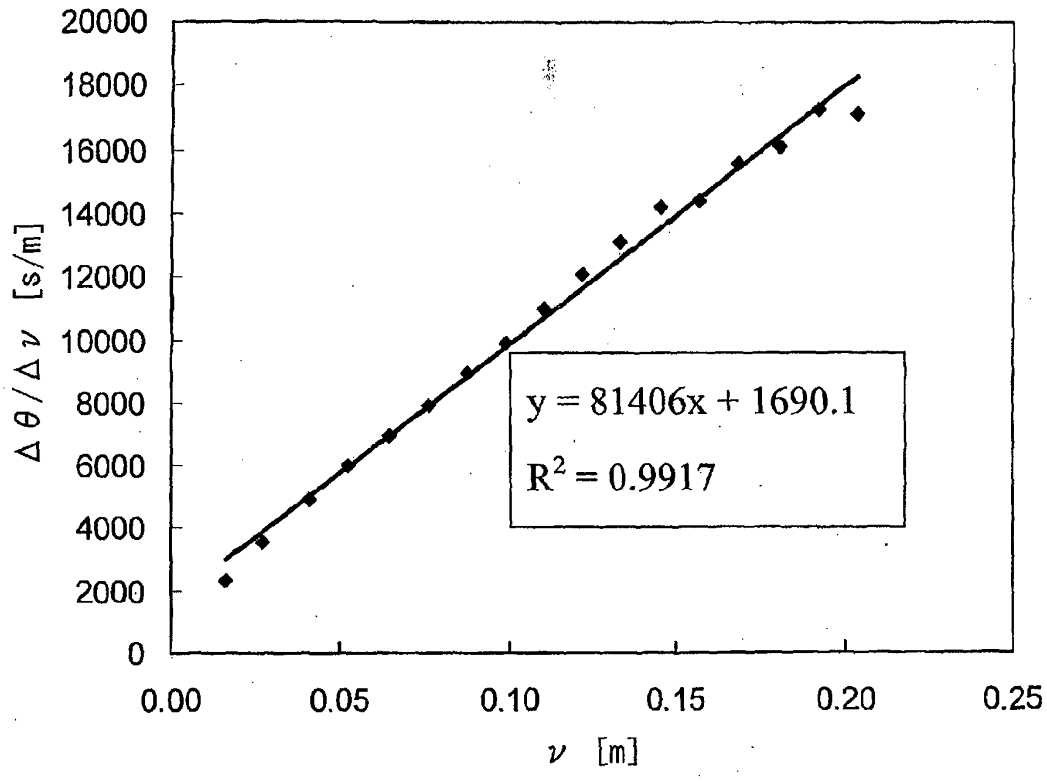
20000
18000
16000
14000
12000
10000
8000
6000
4000
2000
0
Δθ/Δν [s/m]
y = 81406x + 1690.1
R² = 0.9917
0.00
0.05
0.10
0.15
0.20
0.25
ν [m]

[Figure 2]

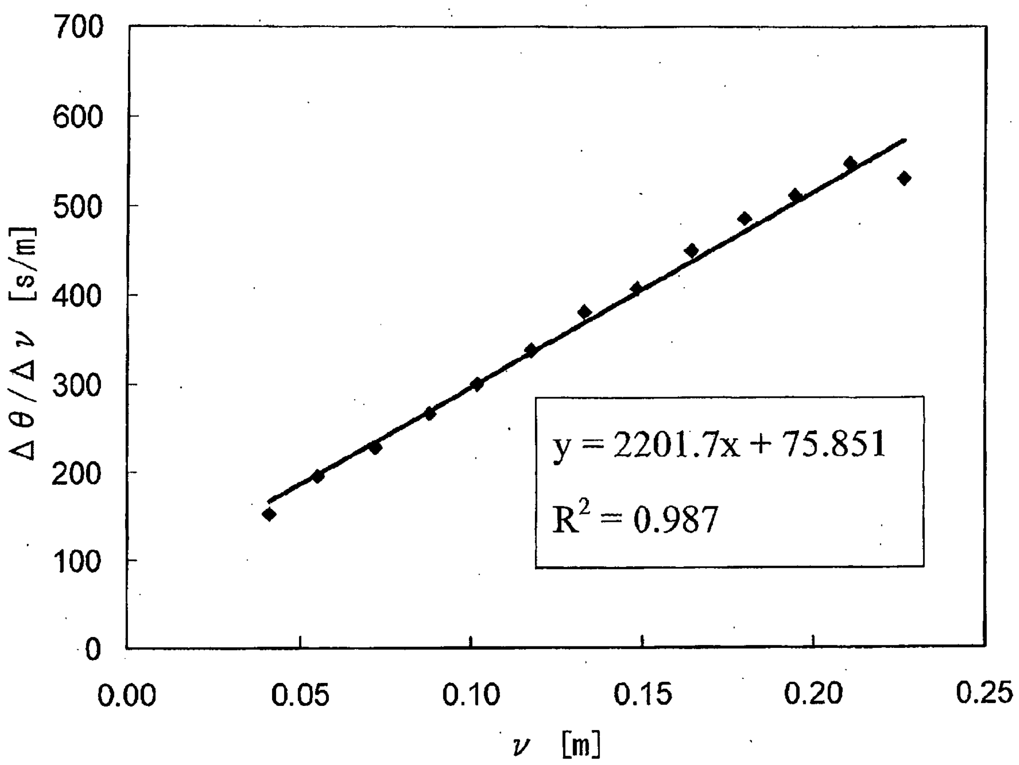


[Figure 3]

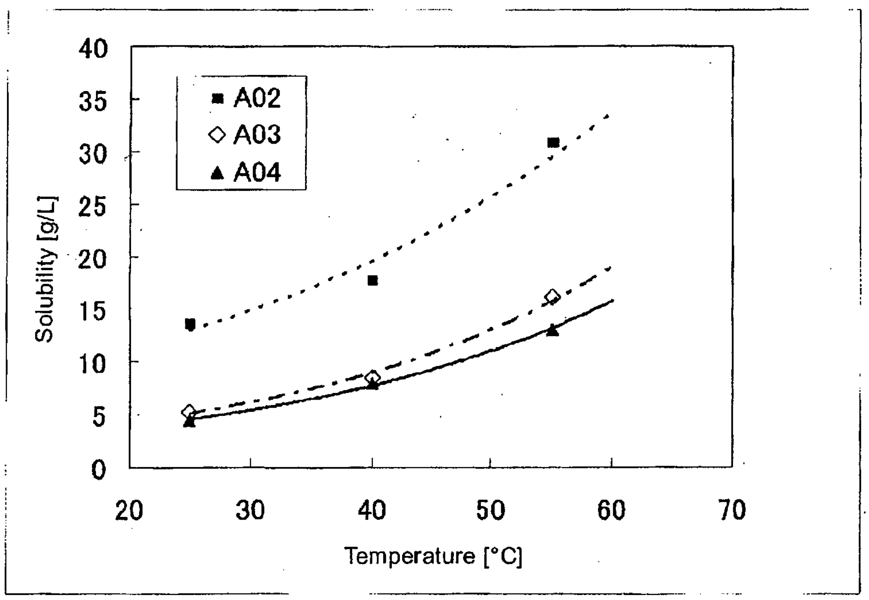

[Figure 4]

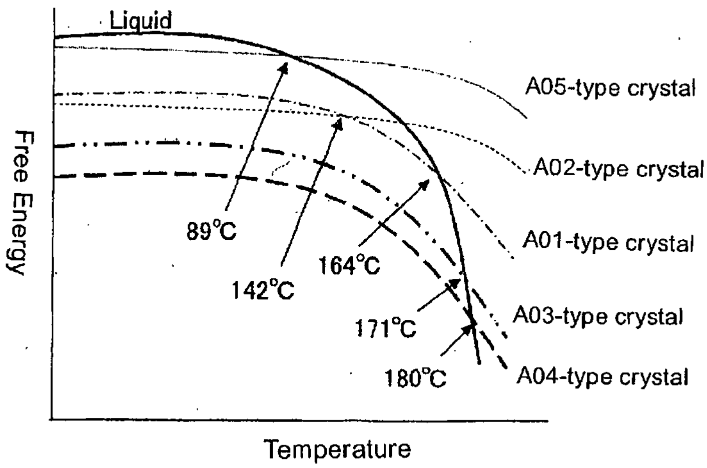
Liquid
A05-type crystal
A02-type crystal
A01-type crystal
A03-type crystal
A04-type crystal
89°C
142°C
164°C
171°C
180°C
Free Energy
Temperature

[Figure 5]

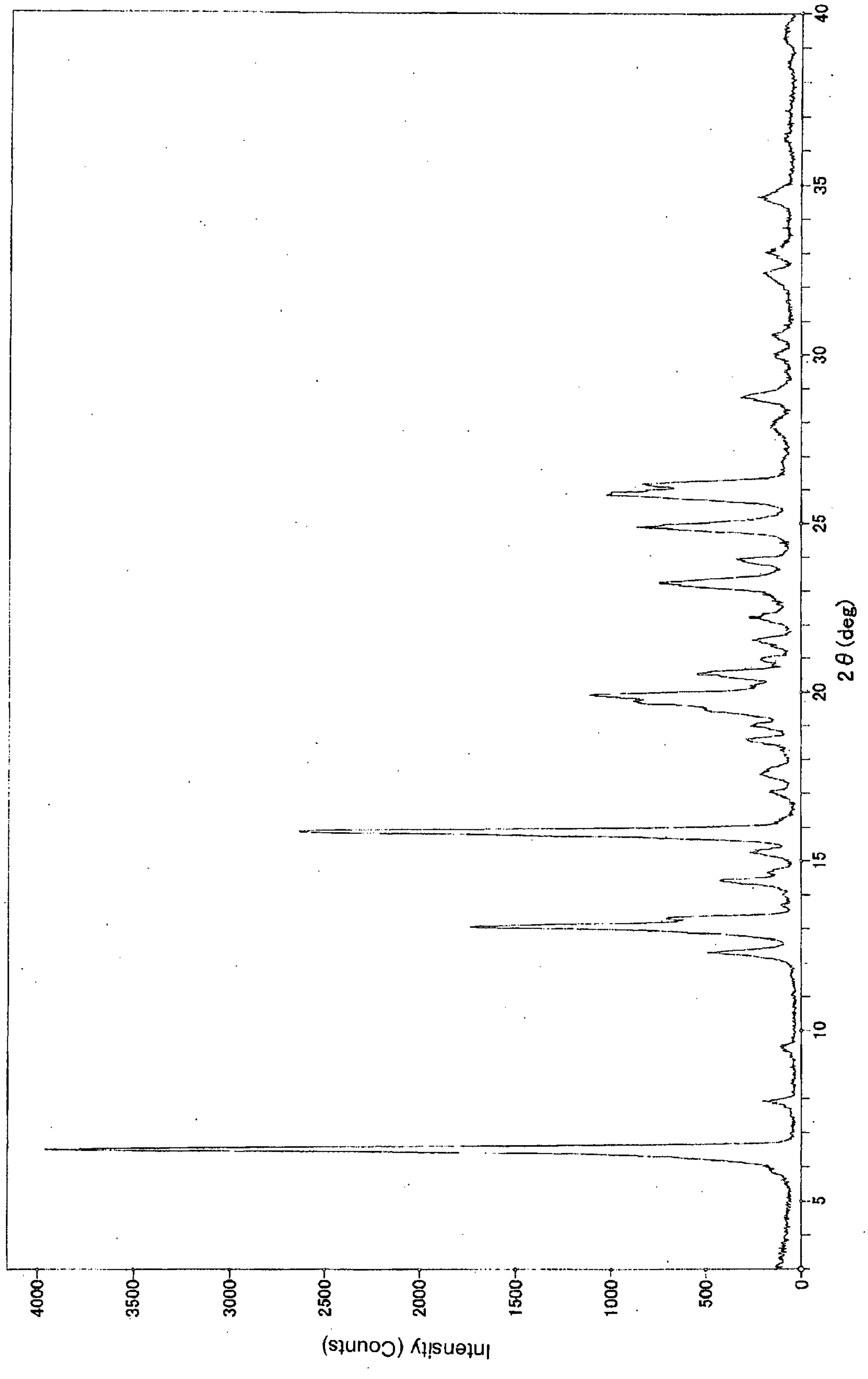
Intensity (Counts)
0
500
1000
1500
2000
2500
3000
3500
4000
2θ (deg)
5
10
15
20
25
30
35
40

[Figure 6]

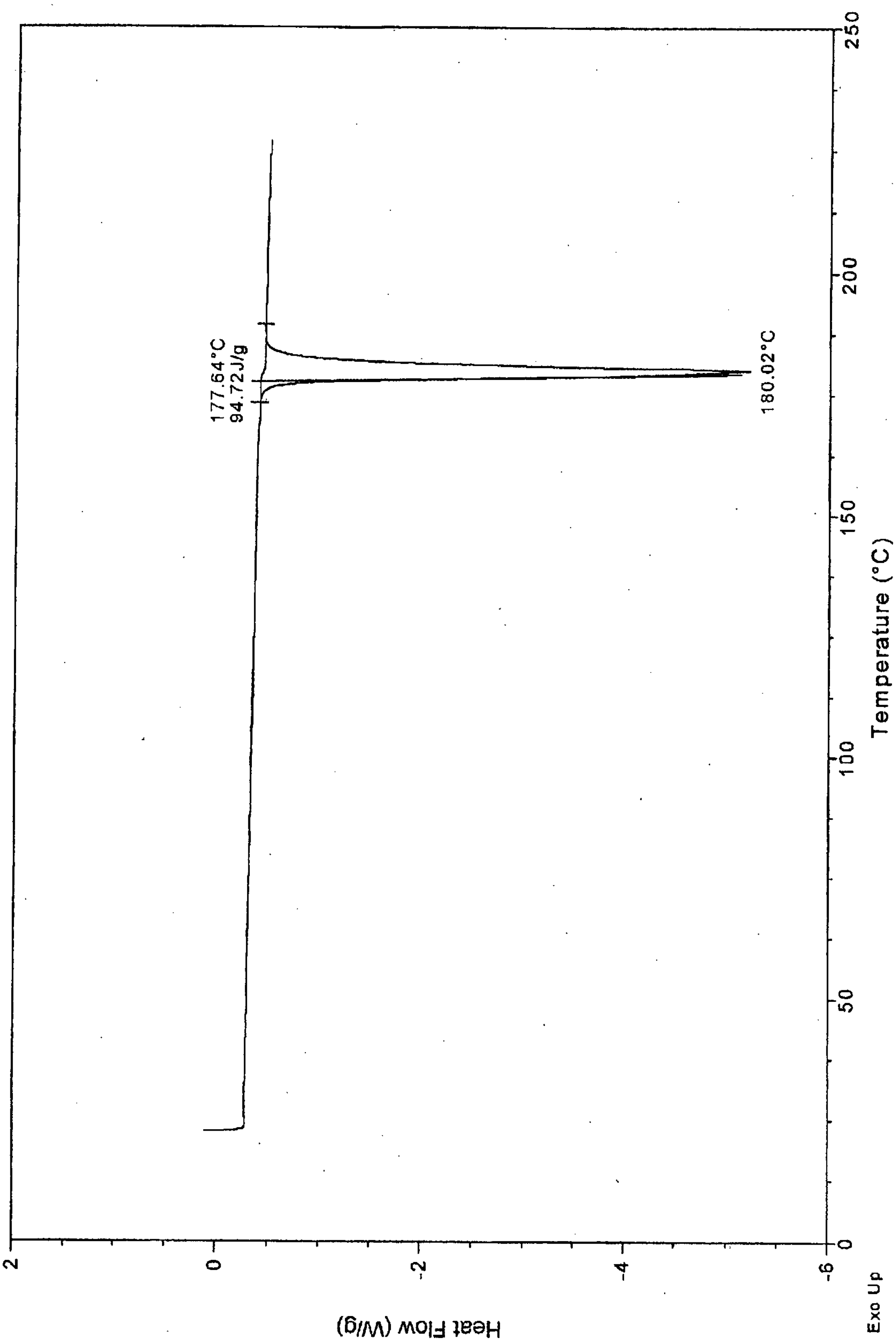
177.64°C
94.72J/g
180.02°C
Temperature (°C)
Heat Flow (W/g)
Exo Up
0
50
100
150
200
250
2
0
-2
-4
-6

[Figure 7]

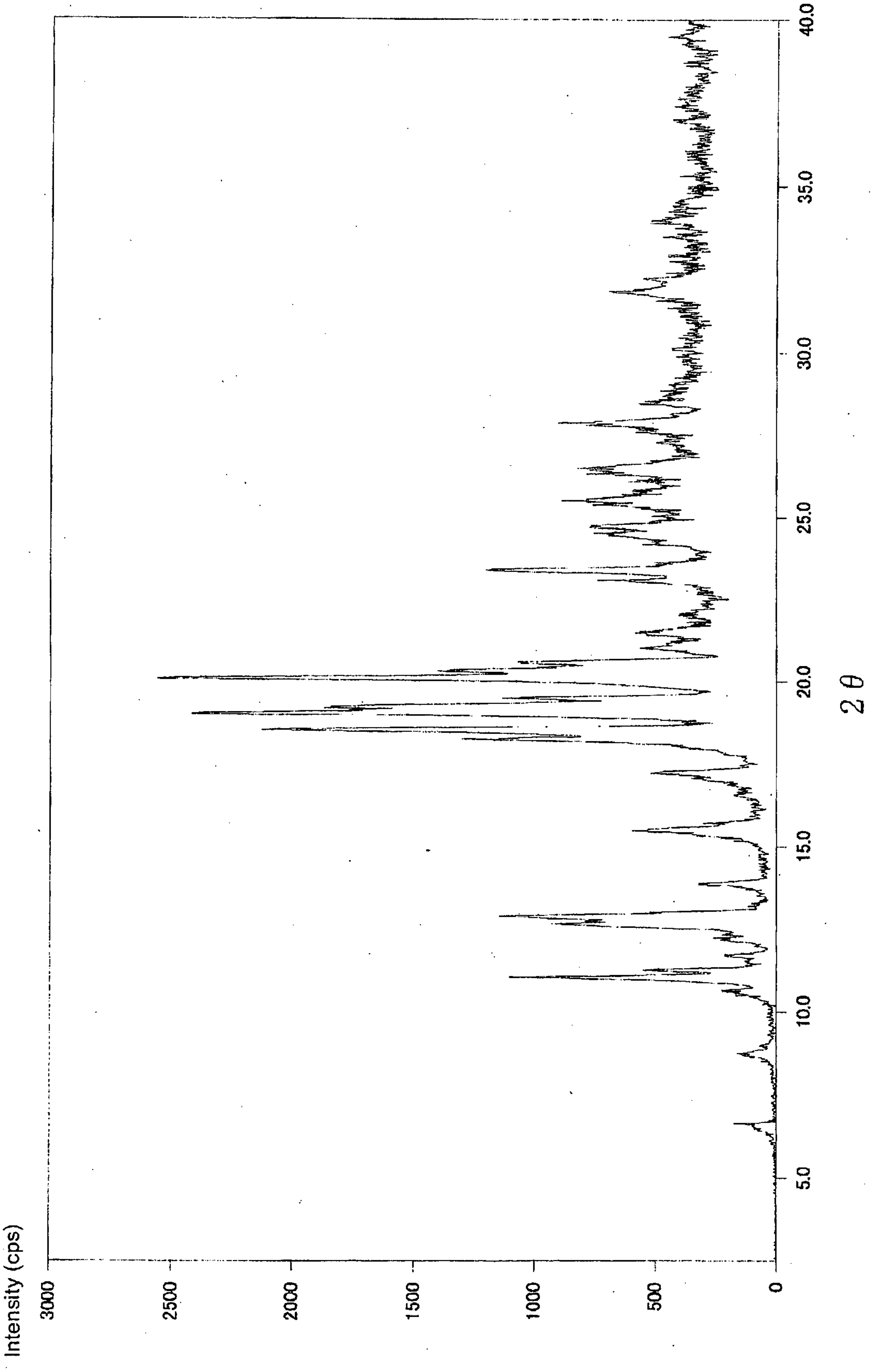
Intensity (cps)
3000
2500
2000
1500
1000
500
0
5.0
10.0
15.0
20.0
25.0
30.0
35.0
40.0
2θ

[Figure 8]

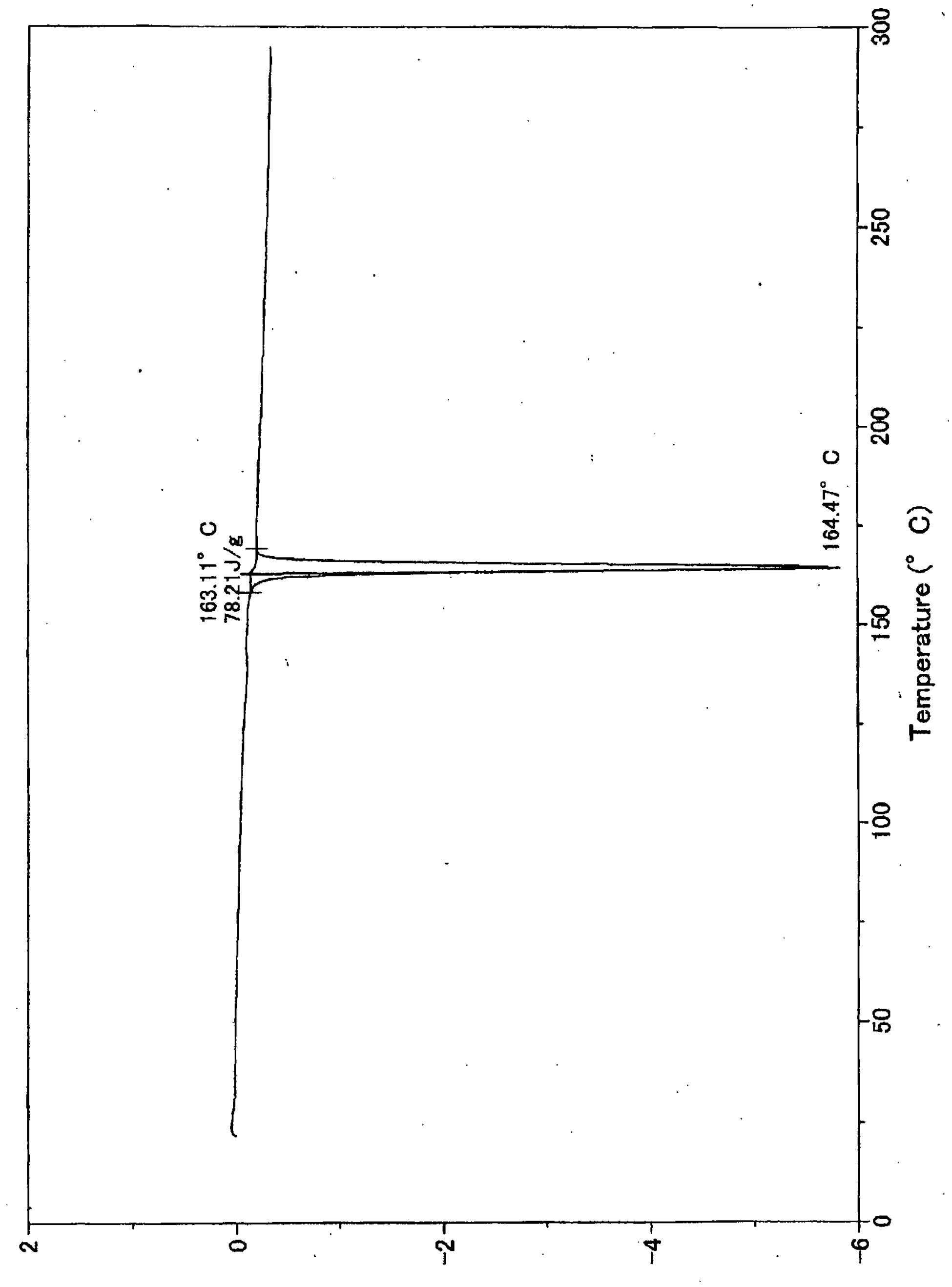

163.11° C
78.21J/g
164.47° C
Temperature (° C)
Heat Flow (W/g)
0
50
100
150
200
250
300
2
0
-2
-4
-6

[Figure 9]

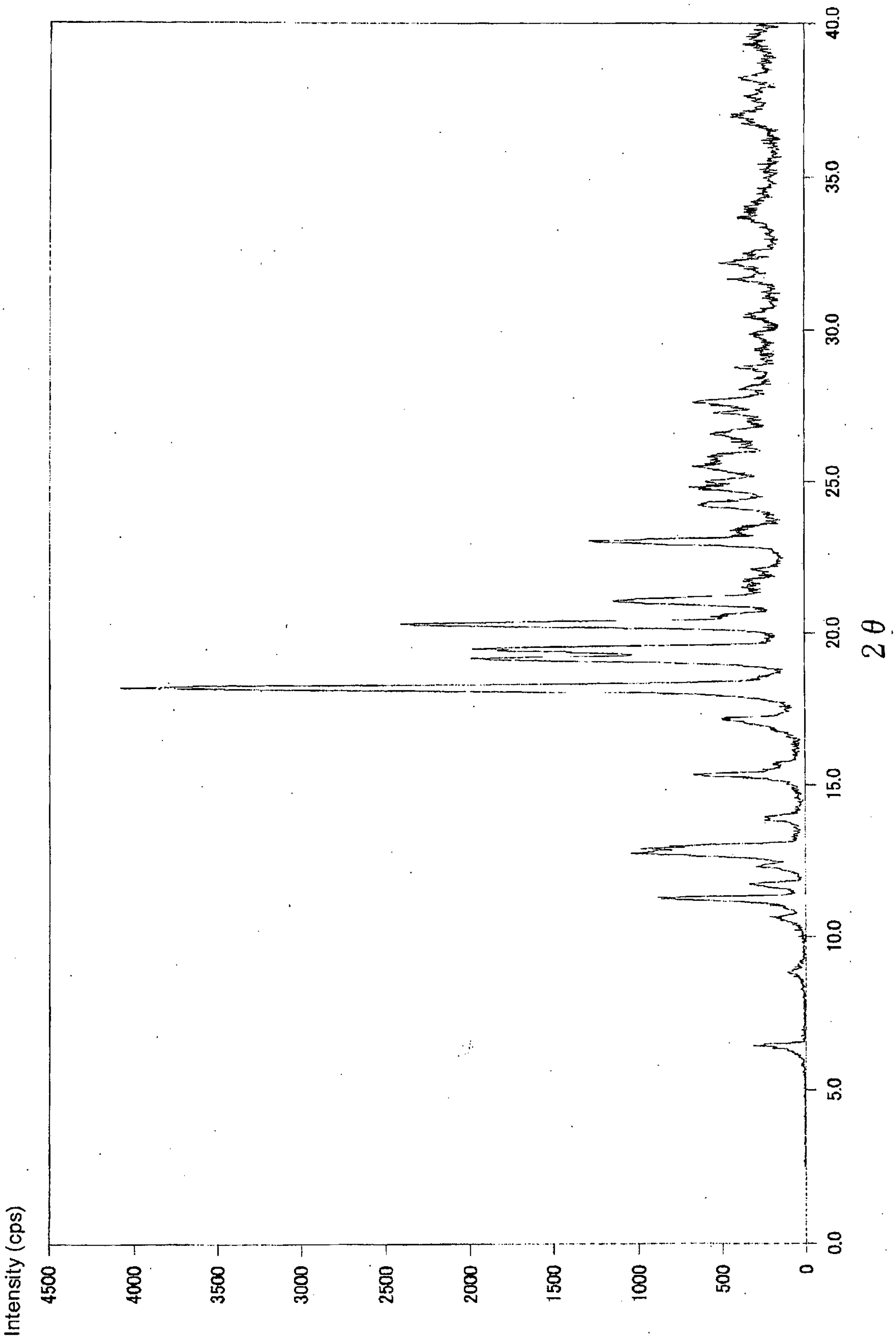
Intensity (cps)
4500
4000
3500
3000
2500
2000
1500
1000
500
0
0.0
5.0
10.0
15.0
20.0
25.0
30.0
35.0
40.0
2θ

[Figure 10]

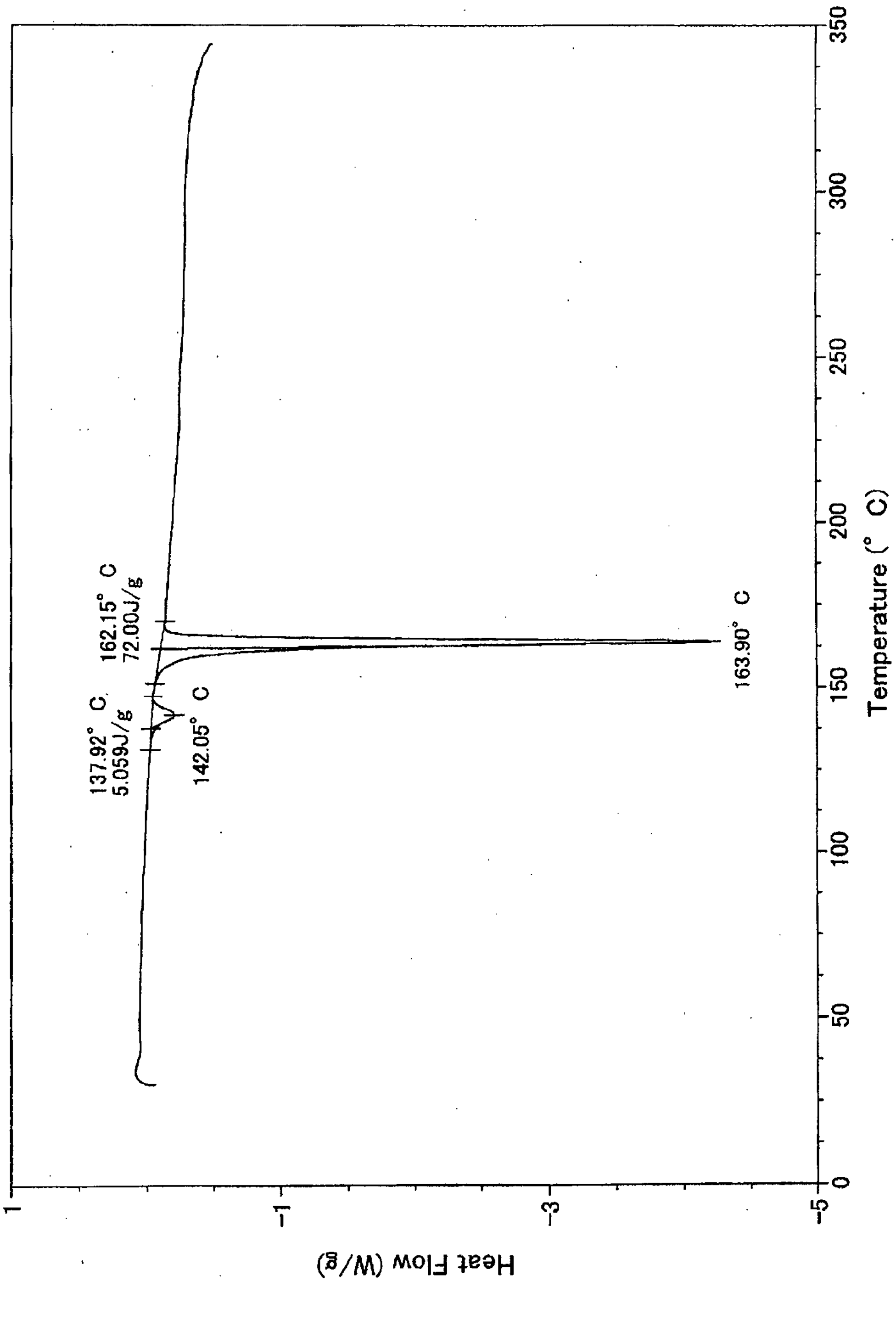
162.15° C
72.00J/g
137.92° C
5.059J/g
142.05° C
163.90° C
Temperature (° C)
0
50
100
150
200
250
300
350
Heat Flow (W/g)
1
-1
-3
-5

[Figure 11]

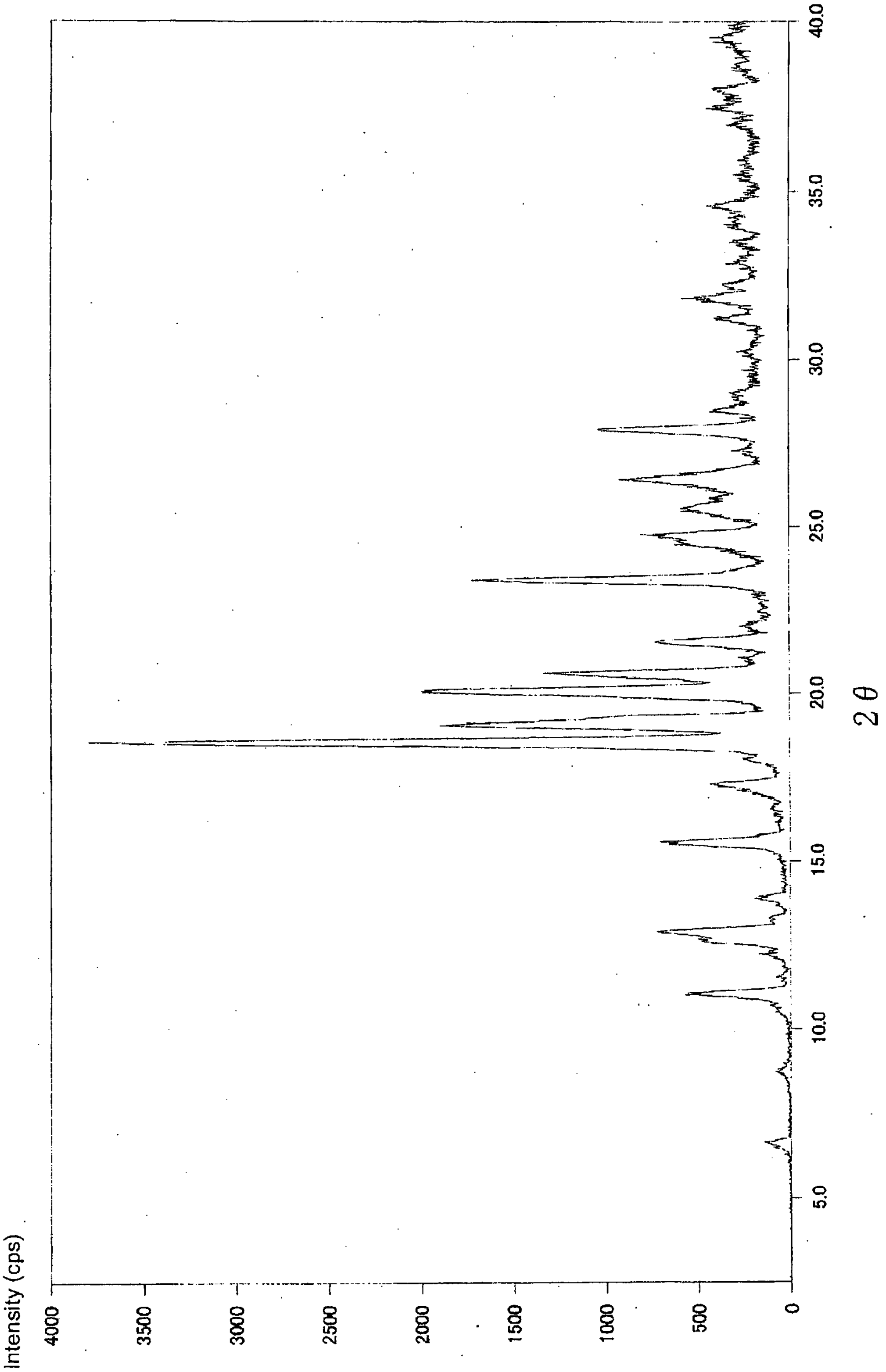
Intensity (cps)
4000
3500
3000
2500
2000
1500
1000
500
0
5.0
10.0
15.0
20.0
25.0
30.0
35.0
40.0
2θ

[Figure 12]

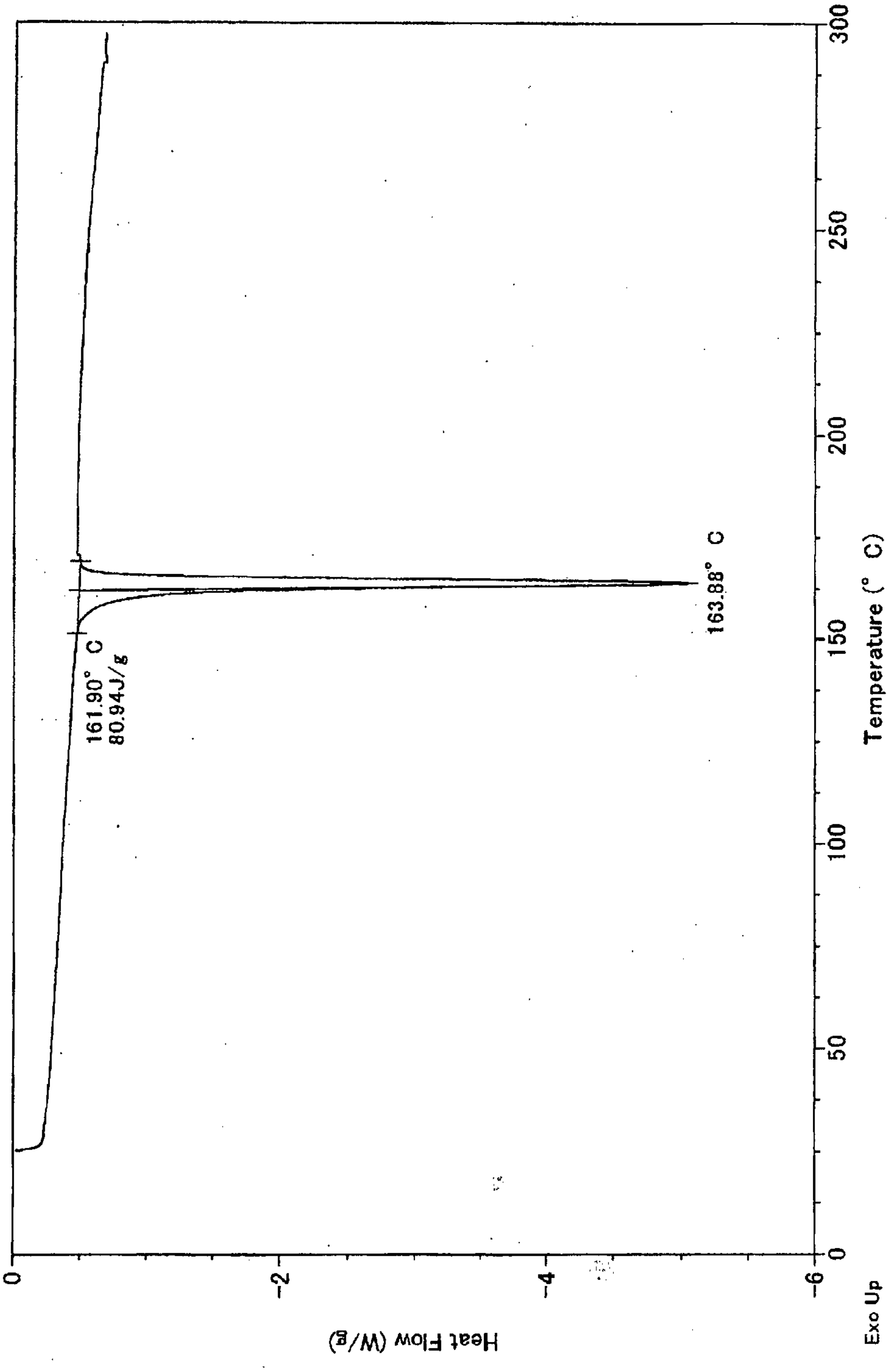
161.90° C
80.94J/g
163.88° C
Temperature (° C)
Heat Flow (W/g)
Exo Up
0
-2
-4
-6
0
50
100
150
200
250
300

[Figure 13]

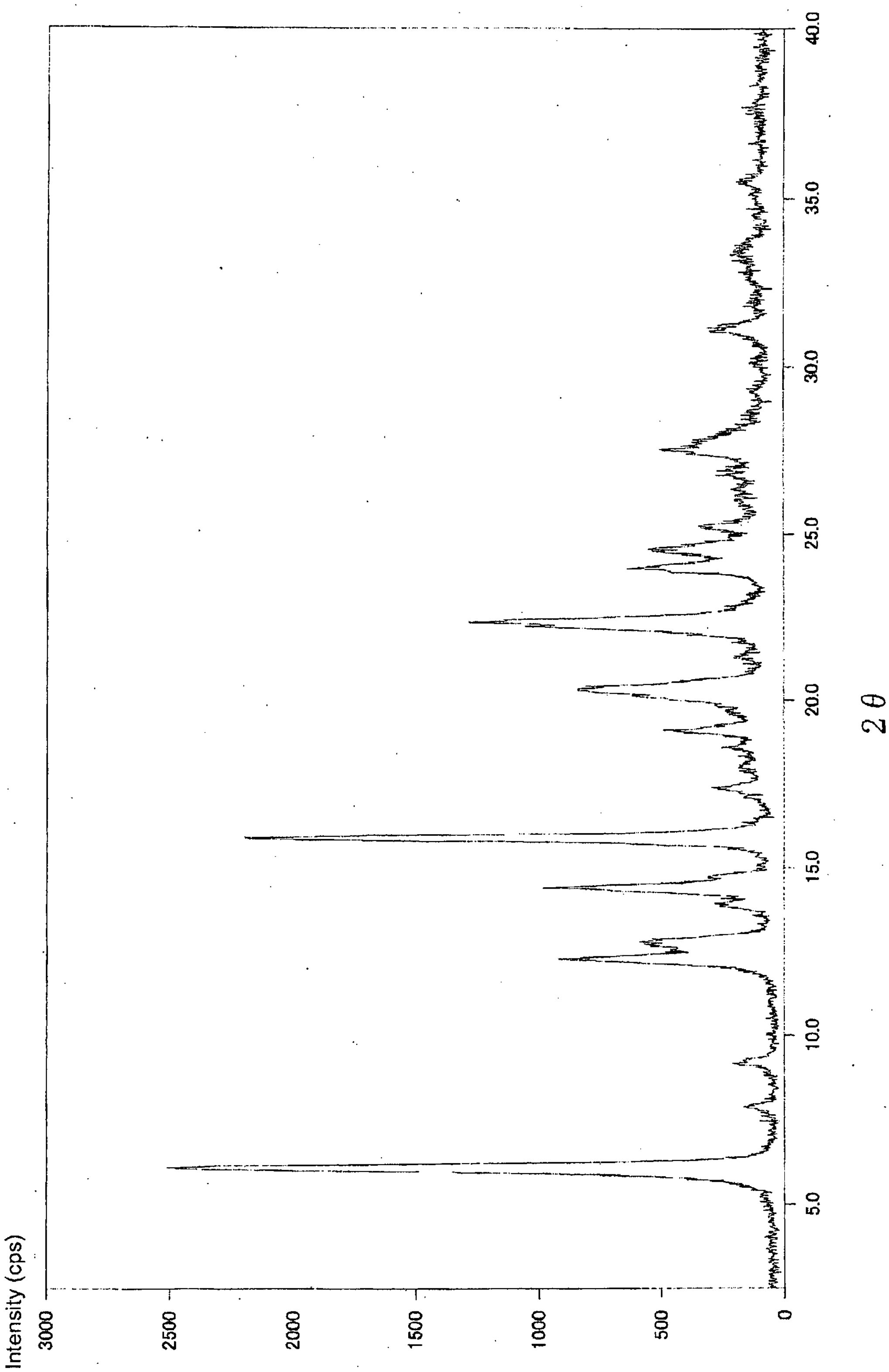
Intensity (cps)
3000
2500
2000
1500
1000
500
0
5.0
10.0
15.0
20.0
25.0
30.0
35.0
40.0
2θ

[Figure 14]

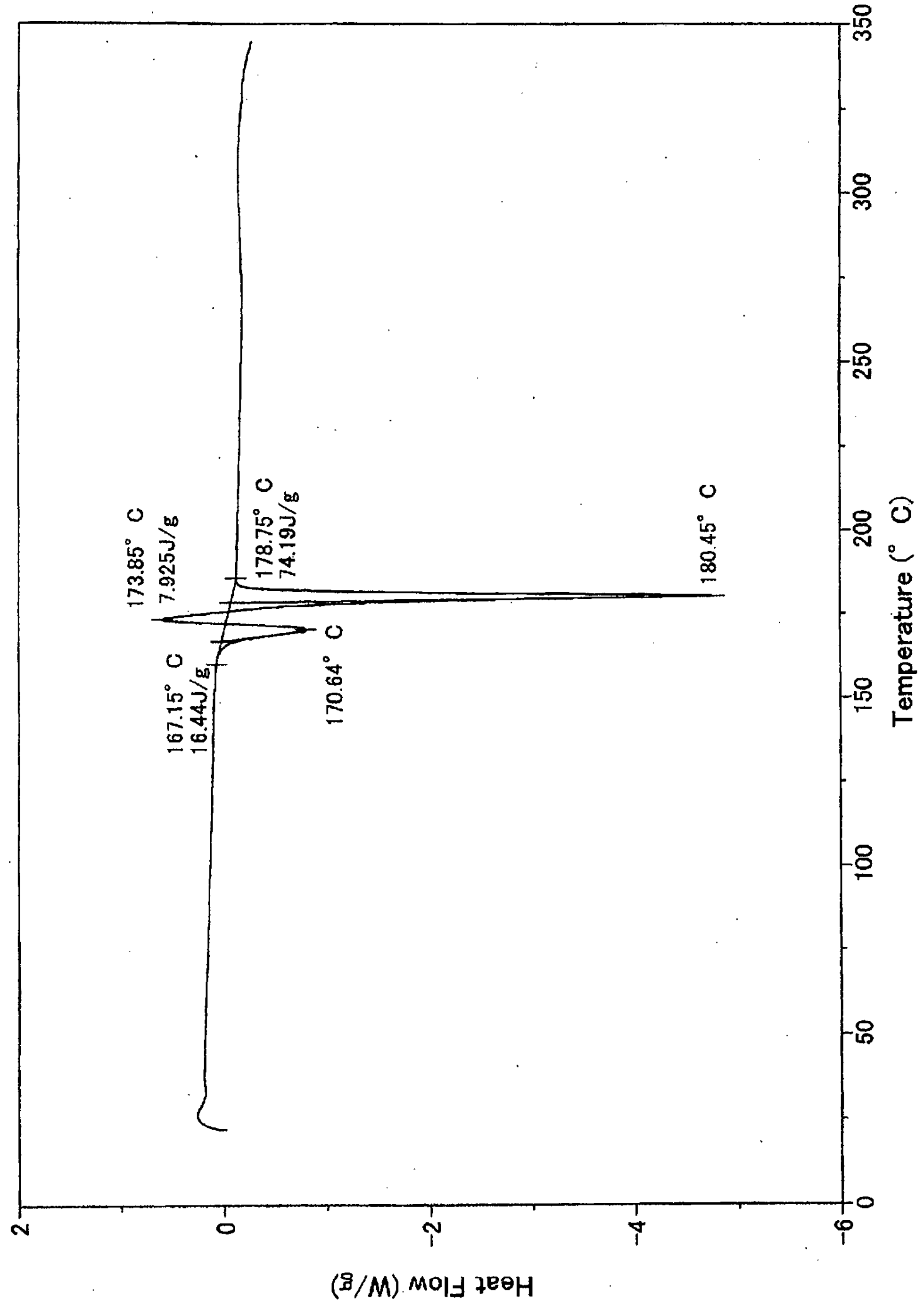
173.85° C
7.925J/g
178.75° C
74.19J/g
180.45° C
167.15° C
16.44J/g
170.64° C
0
50
100
150
200
250
300
350
Temperature (° C)
2
0
-2
-4
-6
Heat Flow (W/g)

[Figure 15]

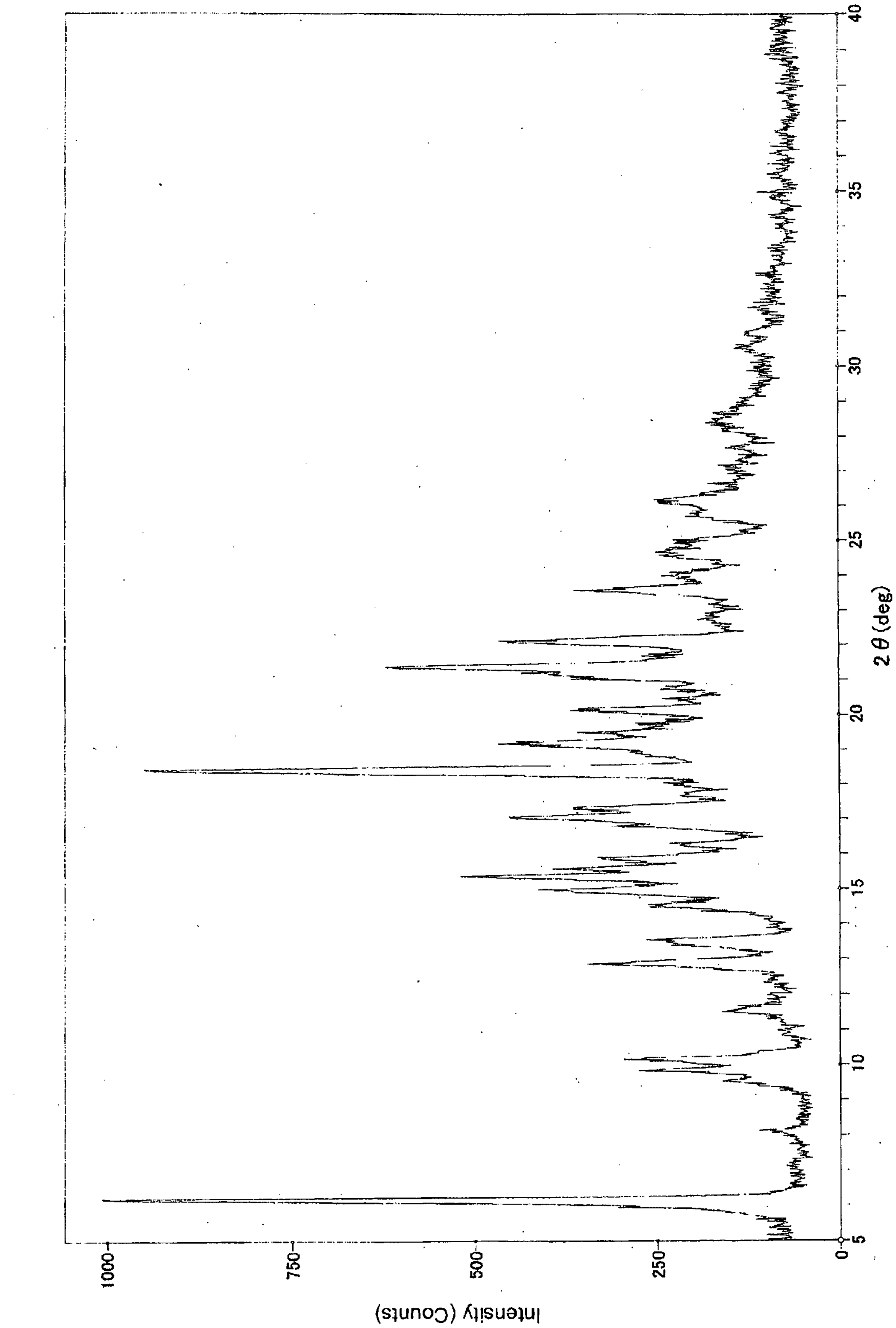

[Figure 16]

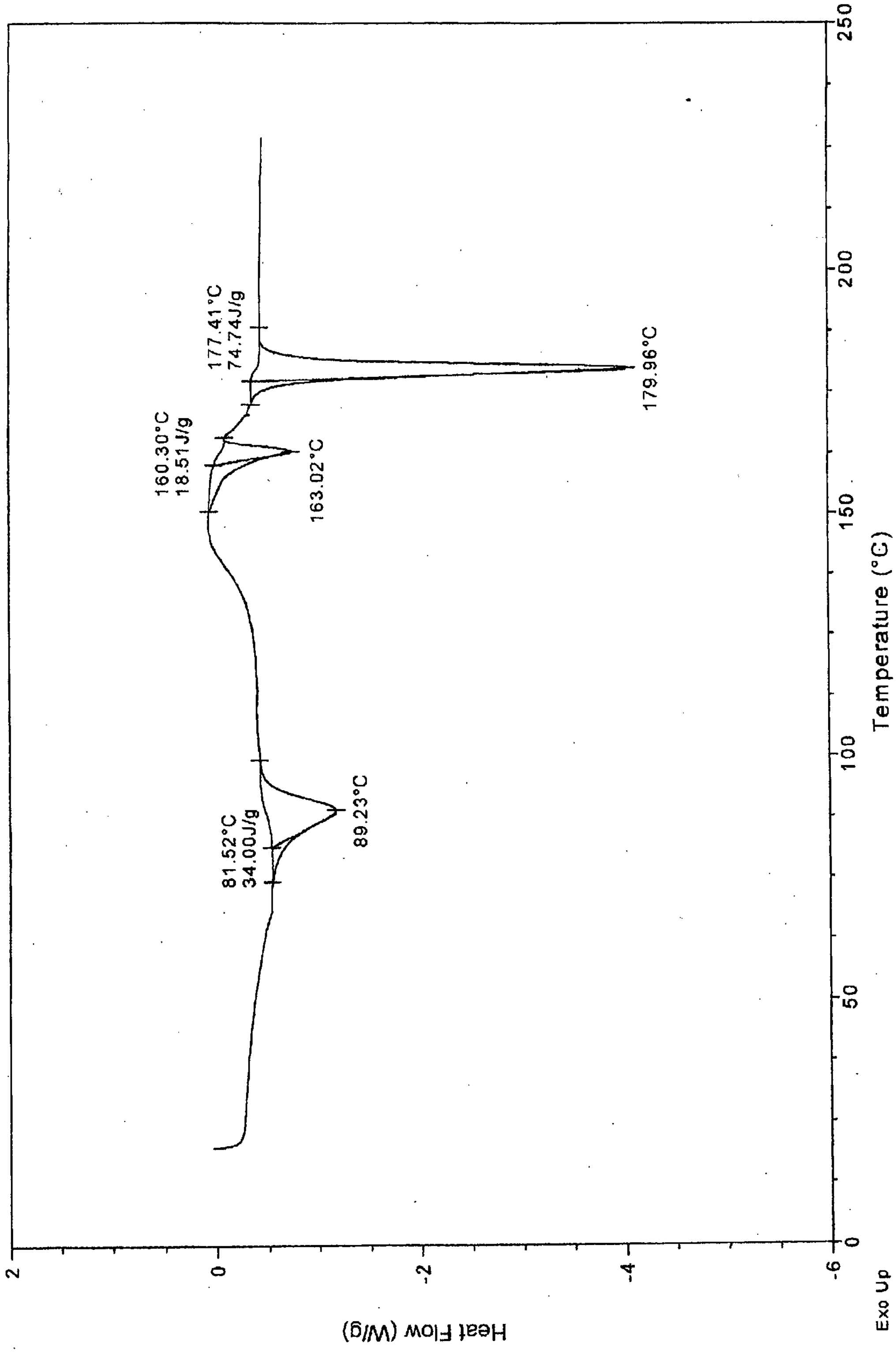
177.41°C
74.74J/g
179.96°C
160.30°C
18.51J/g
163.02°C
81.52°C
34.00J/g
89.23°C
Heat Flow (W/g)
Temperature (°C)
Exo Up
2
0
-2
-4
-6
0
50
100
150
200
250

[Figure 17]

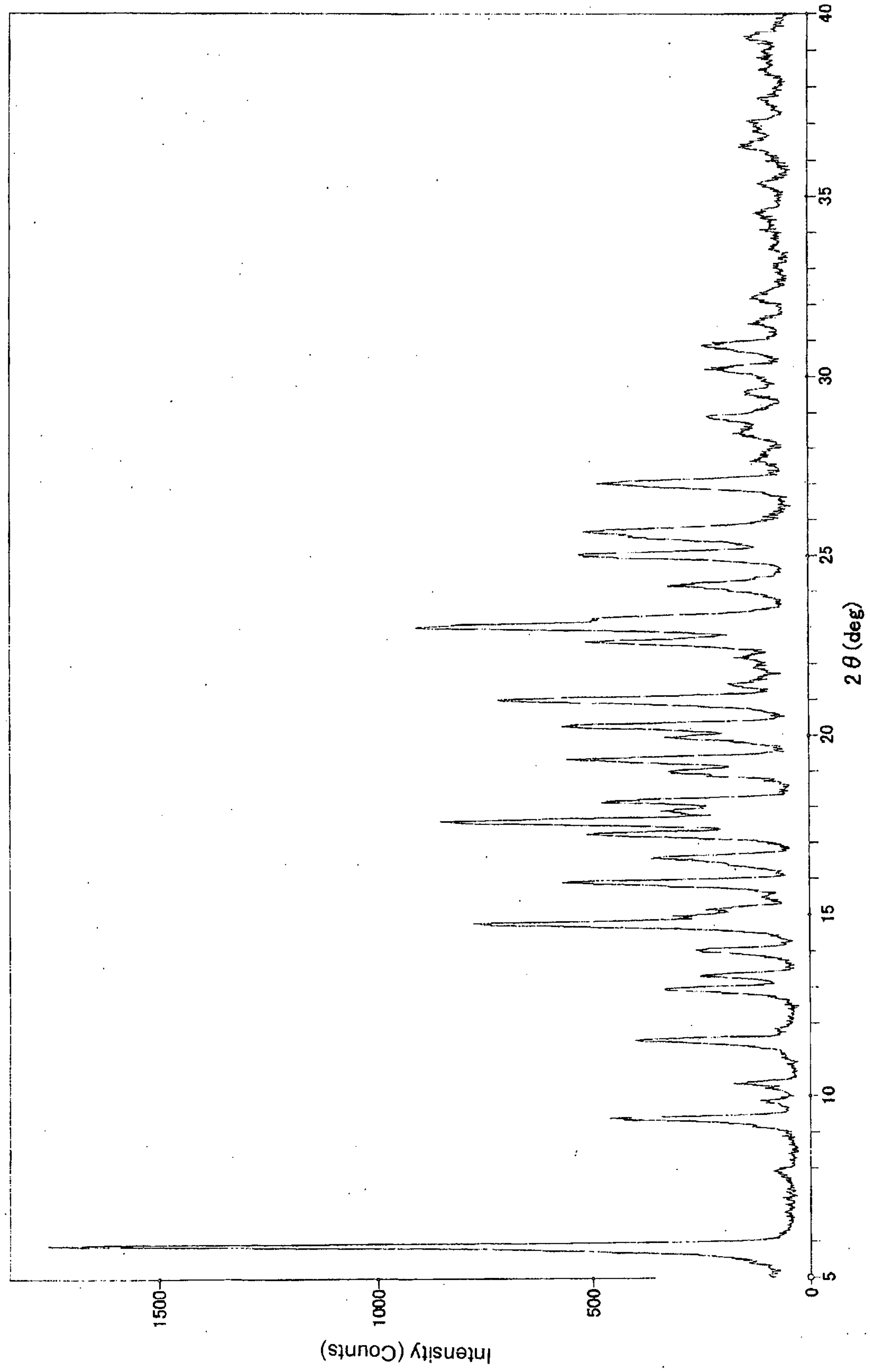

[Figure 18]

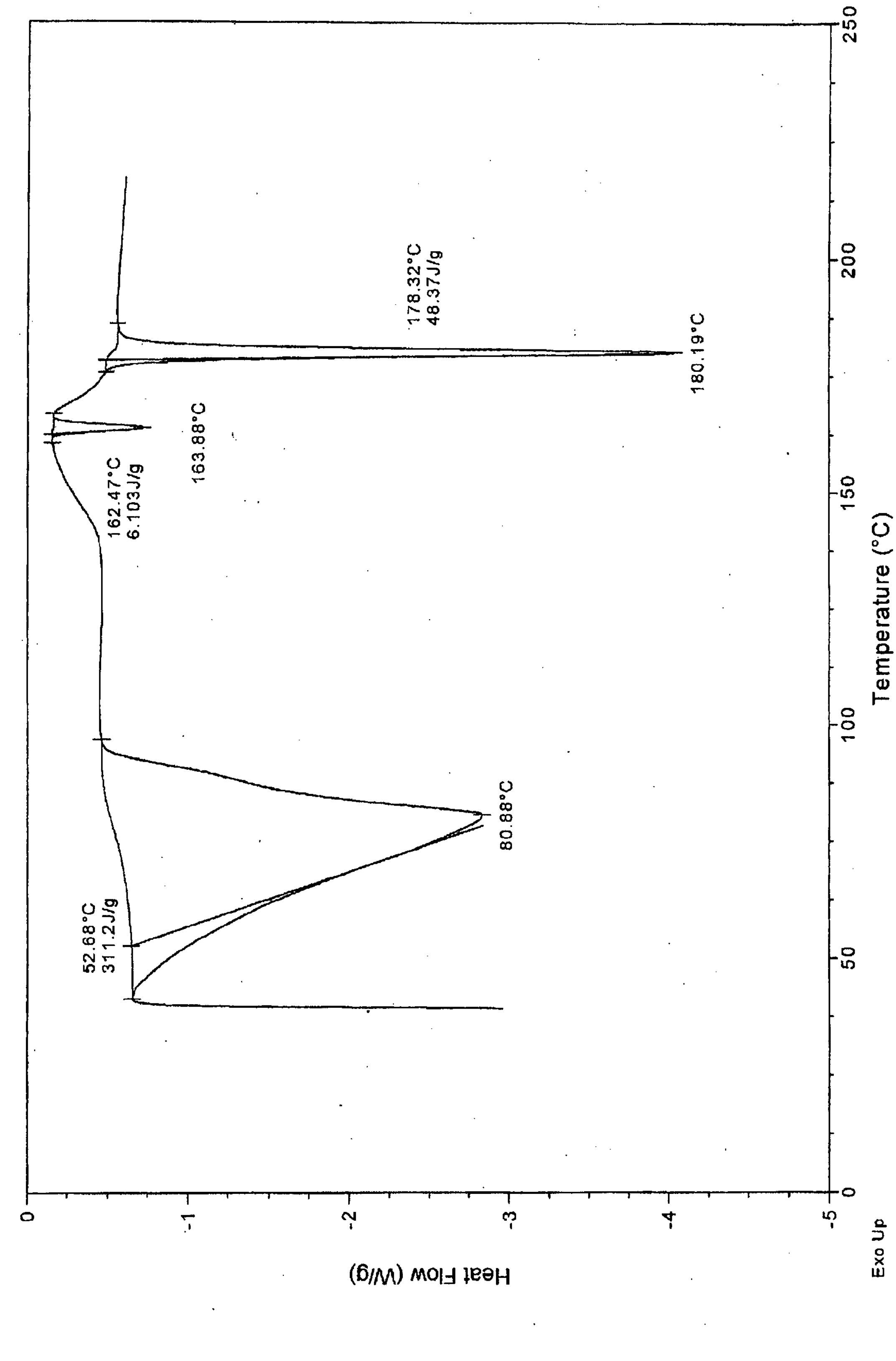
178.32°C
48.37J/g
180.19°C
163.88°C
162.47°C
6.103J/g
80.88°C
52.68°C
311.2J/g
0
50
100
150
200
250
Temperature (°C)
0
-1
-2
-3
-4
-5
Heat Flow (W/g)
Exo Up

**INTERNATIONAL SEARCH REPORT**

International application No.
PCT/JP2011/061156

A. CLASSIFICATION OF SUBJECT MATTER
*C07D401/12*(2006.01)i, *A61K31/53*(2006.01)i, *A61P35/00*(2006.01)i, *A61P43/00* (2006.01)i

According to International Patent Classification (IPC) or to both national classification and IPC

B. FIELDS SEARCHED

Minimum documentation searched (classification system followed by classification symbols)
C07D401/12, A61K31/53, A61P35/00, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2009/008371 A1 (Astellas Pharma Inc.), 15 January 2009 (15.01.2009), example 23 & US 2010/0099658 A & EP 2172461 A1 | 1-8,10 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
28 June, 2011 (28.06.11)

Date of mailing of the international search report
12 July, 2011 (12.07.11)

Name and mailing address of the ISA/
Japanese Patent Office

Authorized officer

Facsimile No.

Telephone No.

Form PCT/ISA/210 (second sheet) (July 2009)

**INTERNATIONAL SEARCH REPORT**

International application No.
PCT/JP2011/061156

**Box No. II Observations where certain claims were found unsearchable (Continuation of item 2 of first sheet)**

This international search report has not been established in respect of certain claims under Article 17(2)(a) for the following reasons:

1. [X] Claims Nos.: 9,11
because they relate to subject matter not required to be searched by this Authority, namely:
The inventions set forth in claims 9 and 11 pertain to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009008371 A